# EUROPÄISCHE PATENTSCHRIFT

(11) **EP 1 370 548 B1**
(45) Veröffentlichungstag und Bekanntmachung des Hinweises auf die Patenterteilung: **01.02.2006**
(21) Anmeldenummer: 02722180.3
(22) Anmeldetag: 06.03.2002
(51) Int. Cl.: C07D 333/34, C07D 409/12, A61K 31/381, A61K 31/341, A61P 9/02, A61P 3/10

(54) **P-THIENYLBENZYL-AMIDE ALS AGONISTEN VON ANGIOTENSIN-(1-7)-REZEPTOREN, VERFAHREN ZU IHRER HERSTELLUNG, IHRE VERWENDUNG UND SIE ENTHALTENDE PHARMAZEUTISCHE PRÄPARATE**
P-THIENYLBENZYL-AMIDES SERVING AS AGONISTS OF ANGIOTENSIN-(1-7)-RECEPTORS, METHOD FOR THE PRODUCTION THEREOF, THEIR USE AND PHARMACEUTICAL PREPARATIONS CONTAINING THE SAME
AMIDES DE P-THIENYLBENZYLE SERVANT D'AGONISTES DES RECEPTEURS DE L'ANGIOTENSINE (1-7), LEUR PROCEDE DE PRODUCTION, LEUR UTILISATION ET PREPARATIONS PHARMACEUTIQUES LES RENFERMANT

(30) Priorität: 14.03.2001 DE 10112041
(43) Veröffentlichungstag der Anmeldung: 17.12.2003
(73) Patentinhaber: Sanofi-Aventis Deutschland GmbH, 65929 Frankfurt am Main (DE)
(72) Erfinder: HEITSCH, Holger, 55252 Mainz-Kastel (DE)
(86) Internationale Anmeldenummer: PCT/EP2002/002422
(87) Internationale Veröffentlichungsnummer: WO 2002/072569

(56) Entgegenhaltungen:
- EP-A- 0 512 675
- EP-A- 0 513 979
- WO-A-00/68226
- GB-A- 2 281 298

## Beschreibung

Die Erfindung betrifft p-Thienylbenzyl-Amide der Formel (I), 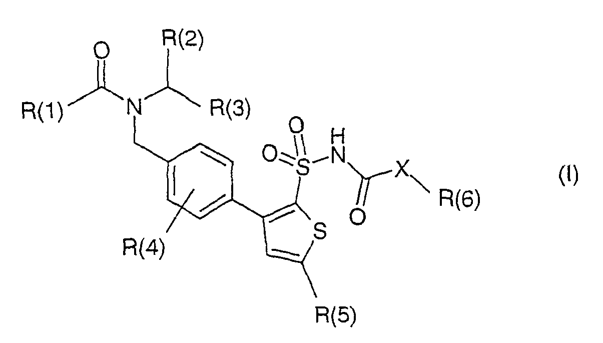
in der R(1), R(2), R(3), R(4), R(5), R(6) und X die unten angegebene Bedeutung haben. Die Verbindungen der Formel (I) stellen potente Agonisten von Angiotensin-(1-7)-Rezeptoren dar. Aufgrund der durch die Verbindungen der Formel (I) hervorgerufenen Stimulation dieser Rezeptoren und der damit an Endothelzellen verbundenen Produktion und Freisetzung der vasorelaxierenden und kardioprotektiven Botenstoffe cyclisches Guanosinmonophoshat (cGMP) und Stickstoffmonoxid (NO) eignen sich die Verbindungen der Formel (I) als wertvolle Arzneimittelwirkstoffe zur Behandlung und Prävention von Bluthochdruck, Herzhypertrophie, Herzinsuffizienz, koronaren Herzerkrankungen wie der Angina Pectoris und einer endothelialen Dysfunktion bzw. endothelialer Schädigungen, z.B. als Folge atheriosklerotischer Prozesse oder beim Diabetis mellitus.

In der PCT-Anmeldung WO-0068226 sind 1-(p-Thienylbenzyl)-Imidazole als Agonisten von Angiotensin-(1-7)-Rezeptoren zur Behandlung und/oder Prophylaxe von Bluthochdruck, Herzhypertrophie, Herzinsuffizienz und einer endothelialen Dysfunktion bzw. endothelialer Schädigungen beschrieben.

In Hinblick auf die vielfältigen Anwendungsmöglichkeiten von ANG-(1-7)-Rezeptor-Agonisten als Arzneimittel und den damit verbundenen Anforderungen an deren Eigenschaften besteht ein Bedarf an weiteren ANG-(1-7)-Rezeptoren-Agonisten mit einem günstigen Wirk-, Selektivitäts- beziehungsweise pharmako-dynamischen bzw. pharmako-kinetischen Eigenschafts-Profil.

Überraschend wurde gefunden, daß p-Thienylbenzyl-Amide der Formel (I) eine ausgeprägte Wirkung auf Angiotensin-(1-7)-Rezeptoren haben und die biologische Wirkung des Effektorhormons Angiotensin-(1-7) mimikrieren.

Ein Gegenstand der Erfindung sind somit Verbindungen der Formel (I), 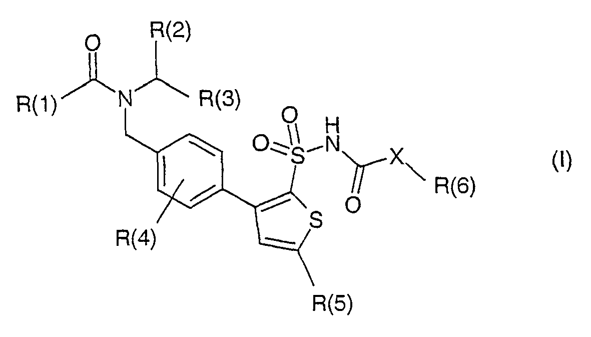
in denen die angeführten Reste die folgende Bedeutung haben:
- R(1): 1. (C₁-C₅)-Alkyl, unsubstituiert oder substituiert mit einem Rest aus der Reihe NH₂, Halogen, O-(C₁-C₃)-Alkyl, CO-O-(C₁-C₃)-Alkyl und CO₂H;
2. (C₃-C₈)-Cycloalkyl;
3. (C₁-C₃)-Alkyl-(C₃-C₈)-Cycloalkyl;
4. (C₆-C₁₀)-Aryl, unsubstituiert oder substituiert mit einem Rest aus der Reihe Halogen und O-(C₁-C₃)-Alkyl;
5. (C₁-C₃)-Alkyl-(C₆-C₁₀)-Aryl, wobei der Arylrest unsubstituiert oder substituiert ist mit einem Rest aus der Reihe Halogen und O-(C₁-C₃)-Alkyl;
6. (C₁-C₅)-Heteroaryl; und
7. (C₁-C₃)-Alkyl-(C₁-C₅)-Heteroaryl;
- R(2): 1. Wasserstoff;
2. (C₁-C₆)-Alkyl, unsubstituiert oder substituiert mit einem Rest aus der Reihe Halogen und O-(C₁-C₃)-Alkyl;
3. (C₃-C₈)-Cycloalkyl;
4. (C₁-C₃)-Alkyl-(C₃-C₈)-Cycloalkyl;
5. (C₆-C₁₀)-Aryl, unsubstituiert oder substituiert mit einem Rest aus der Reihe Halogen, O-(C₁-C₃)-Alkyl und CO-O-(C₁-C₃)-Alkyl; und
6. (C₁-C₃)-Alky)-(C₆-C₁₀)-Aryl, unsubstituiert oder substituiert mit einem Rest aus der Reihe Halogen und O-(C₁-C₃)-Alkyl;
- R(3): 1. Wasserstoff;
2. COOH; und
3. COO-(C₁-C₄)-Alkyl;
- R(4): 1. Wasserstoff;
2. Halogen; und
3. (C₁-C₄)-Alkyl;
- R(5): 1. Wasserstoff und
2. (C₁-C₆)-Alkyl;
- R(6): 1. Wasserstoff;
2. (C₁-C₆)-Alkyl;
3. (C₁-C₃)-Alkyl-(C₃-C₈)-Cycloalkyl; und
4. (C₂-C₆)-Alkenyl;
- X: 1. Sauerstoff und
2. NH;

in allen ihren stereoisomeren Formen und Mischungen davon in allen Verhältnissen, und ihre physiologisch verträglichen Salze.

Der Begriff Alkyl umfasst, sofern nicht anders angegeben, geradkettige oder verzweigte gesättigte Kohlenwasserstoffreste. Dies gilt auch für davon abgeleitete Substituenten wie Alkoxy oder den Resten SO₂NHCOO-Alkyl und SO₂NHCONH-Alkyl. Beispiele für Alkylreste sind Methyl, Ethyl, n-Propyl, Isopropyl, Isobutyl, n-Butyl, n-Hexyl, Isohexyl, n-Heptyl. Beispiele für Alkoxy sind Methoxy, Ethoxy, Propoxy wie n-Propoxy und Isopropoxy.

Alkenyl steht für einfach oder mehrfach ungesättigte Kohlenwasserstoffreste, in denen sich die Doppelbindungen in beliebigen Positionen befinden können. Beispiele für Alkenyl sind Vinyl, Prop-2-enyl (Allyl), Prop-1-enyl, und Butenyl.

Beispiele für Cycloalkyl sind Cyclopropyl, Cyclobutyl, Cyclopentyl, Cyclohexyl, Cycloheptyl und Cyclooctyl.

Halogen steht für Fluor, Chlor, Brom oder Jod, vorzugsweise für Chlor oder Fluor.

Als Beispiele für Arylreste seien Phenyl oder Naphthyl (1- bzw. 2-Naphthyl) genannt.

In substituierten Arylresten können sich die Substituenten in beliebigen Positionen zueinander befinden.

Unter Heteroaryl werden Reste von monocyclischen 5-gliedrigen oder 6-gliedrigen aromatischen Ringsystemen verstanden. Sie können aufgefaßt werden als von Cyclopentadienyl und Phenyl durch Ersatz einer oder zwei CH-Gruppen und/oder CH₂₋Gruppen durch S, O, N, NH (oder einen Substituenten tragendes N wie z.B. N-CH₃) abgeleitete Reste, wobei das aromatische Ringsystem erhalten bleibt oder ein aromatisches Ringsystem ausgebildet wird. Neben den ein, zwei, drei oder vier Ring-Heteroatomen enthalten sie ein, zwei, drei, vier oder fünf Ring-Kohlenstoffatome ((C₁₋C₅)-Heteroaryl). Beispiele für Heteroaryl sind insbesondere Furanyl, Thienyl, Pyrrolyl, Imidazolyl, Triazolyl, Tetrazolyl, Pyrazolyl, Oxazolyl, Isoxazolyl, Thiazolyl, Isothiazolyl, Pyridyl, Pyrazinyl, Pyrimidyl. Ein Heteroarylrest kann über jedes geeignete Kohlenstoffatom gebunden sein.

R(1) steht bevorzugt für
1. (C₁-C₅)-Alkyl insbesondere Methyl; oder
2. (C₁-C₅)-Alkyl, substituiert mit einem Rest aus der Reihe CO-O-(C₁-C₃)-Alkyl und CO₂H; insbesondere Carboxypropionyl und Methoxycarbonylpropionyl; oder
3. (C₁-C₃)-Alkyl-(C₃-C₈)-Cycloalkyl, insbesondere Cyclohexylmethyl; oder
4. (C₆-C₁₀)-Aryl, insbesondere Phenyl; oder
5. (C₁-C₅)-Heteroaryl, insbesondere Furanyl.

R(2) steht bevorzugt für
1. Wasserstoff; oder
2. (C₁-C₆)-Alkyl, insbesondere Isopropyl; oder
3. (C₁-C₆)-Alkyl substituiert mit O-(C₁-C₃)-Alkyl, insbesondere Methoxymethylen; oder
4. (C₃-C₈)-Cycloalkyl insbesondere Cyclopropyl und Cyclohexyl; oder
5. (C₆-C₁₀)-Aryl, insbesondere Phenyl.

R(3) steht bevorzugt für
1. COOH; oder
2. COO-(C₁-C₄)-Alkyl, insbesondere Methoxycarbonyl und Ethoxycarbonyl.

R(4) steht bevorzugt für Wasserstoff.

R(5) steht bevorzugt für (C₁-C₆)-Alkyl, insbesondere Isobutyl.

R(6) steht bevorzugt für (C₁-C₆)-Alkyl, insbesondere Methyl, Ethyl und Butyl.

Die vorliegende Erfindung umfaßt alle stereoisomeren Formen der Verbindungen der Formel (I). In den Verbindungen der Formel (I) enthaltende Asymmetriezentren können alle unabhängig voneinander die S- oder die R-Konfiguration aufweisen. Zur Erfindung gehören alle möglichen Enantiomeren und Diastereomeren, ebenso Mischungen von zwei oder mehr diastereomeren Formen, zum Beispiel Mischungen aus Enantiomeren und/oder Diastereomeren, in allen Verhältnissen. Bei Vorliegen einer cis/trans-Isomerie sind sowohl die cis- als auch die trans-Form und Gemische dieser Formen in allen Verhältnissen Gegenstand der Erfindung. Die Erfindung umfaßt auch alle tautomeren Formen der Verbindungen der Formel (I).

Unter physiologisch verträglichen Salzen von Verbindungen der Formel (I) versteht man sowohl deren anorganische als auch organische Salze, wie sie in Remington's Pharmaceutical Sciences (A.R. Gennard, Editor, Mack Publishing Co, Easton PA, 17. Auflage, Seite 14-18 (1985)) beschrieben sind. Aufgrund der physiologischen und chemischen Stabilität und der Löslichkeit sind für saure Gruppen unter anderen Natrium-, Kalium-, Calcium-, Magnesium- und Ammonium-Salze bevorzugt; Umsetzungen von Verbindungen der Formel (I) mit Basen zur Herstellung der Salze werden im allgemeinen gemäß üblichen Vorgehensweisen in einem Lösung- oder Verdünnungsmittel durchgeführt.

Die vorliegende Erfindung umfaßt weiterhin Solvate von Verbindungen der Formel (I), zum Beispiel Hydrate oder Addukte mit Alkoholen.

Bevorzugt sind Verbindungen der Formel (I), in denen
- R(1): 1. (C₁-C₅)-Alkyl, unsubstituiert oder substituiert mit einem Rest aus der Reihe NH₂, Halogen, O-(C₁-C₃)-Alkyl, CO-O-(C₁-C₃)-Alkyl und CO₂H;
2. (C₃-C₆)-Cycloalkyl;
3. (C₁-C₃)-Alkyl-(C₃-C₆)-Cycloalkyl;
4. (C₆-C₁₀)-Aryl, unsubstituiert oder substituiert mit einem Rest aus der Reihe Halogen und O-(C₁-C₃)-Alkyl;
5. (C₁-C₃)-Alkyl-(C₆-C₁₀)-Aryl, wobei der Arylrest unsubstituiert oder substituiert ist mit einem Rest aus der Reihe Halogen und O-(C₁-C₃)-Alkyl;
6. (C₃-C₅)-Heteroaryl; und
7. (C₁-C₃)-Alkyl-(C₃-C₅)-Heteroaryl;
- R(2): 1. Wasserstoff;
2. (C₁-C₆)-Alkyl, unsubstituiert oder substituiert mit einem Rest aus der Reihe Halogen und O-(C₁-C₃)-Alkyl;
3. (C₃-C₆)-Cycloalkyl;
4. (C₁-C₃)-Alkyl-(C₃-C₆)-Cycloalkyl;
5. (C₆-C₁₀)-Aryl, unsubstituiert oder substituiert mit einem Rest aus der Reihe Halogen, O-(C₁-C₃)-Alkyl und CO-O-(C₁-C₃)-Alkyl; und
6. (C₁-C₃)-Alkyl-(C₆-C₁₀)-Aryl, unsubstituiert oder substituiert mit einem Rest aus der Reihe Halogen und O-(C₁-C₃)-Alkyl;
- R(3): 1. Wasserstoff;
2. COOH; und
3. COO-(C₁-C₄)-Alkyl;
- R(4): 1. Wasserstoff;
2. Halogen; und
3. (C₁-C₄)-Alkyl;
- R(5): 1. Wasserstoff und
2. (C₁-C₄)-Alkyl;
- R(6): 1. Wasserstoff;
2. (C₁-C₄)-Alkyl;
3. (C₁-C₃)-Alkyl-(C₃-C₆)-Cycloalkyl; und
4. (C₃-C₅)-Alkenyl;
- X: 1. Sauerstoff und
2. NH;

bedeuten in allen ihren stereoisomeren Formen und Mischungen davon in allen Verhältnissen, und ihre physiologisch verträglichen Salze.

Besonders bevorzugt sind Verbindungen der Formel (I), in denen
- R(1): 1. (C₁-C₃)-Alkyl, unsubstituiert oder substituiert mit einem Rest aus der Reihe Fluor, Methoxy, Ethoxy, CO-O-(C₁-C₃)-Alkyl und CO₂H;
2. (C₁-C₃)-Alkyl-Cyclohexyl;
3. Phenyl, substituiert oder unsubstituiert mit einem Rest aus der Reihe Fluor und Methoxy;
4. (C₁-C₃)-Alkyl-Phenyl, wobei der Phenylrest unsubstituiert oder substituiert ist mit einem Rest aus der Reihe Fluor und Methoxy;
5. Furanyl, Thienyl oder Pyridyl;
- R(2): 1. Wasserstoff;
2. (C₁-C₆)-Alkyl, unsubstituiert oder substituiert mit einem Rest aus der Reihe Fluor, Methoxy und Ethoxy;
3. Phenyl, unsubstituiert oder substituiert mit einem Rest aus der Reihe Fluor und Methoxy;
4. (C₁-C₆)-Cycloalkyl;
- R(4): 1. Wasserstoff;
2. Methyl; und
3. Chlor;
- R(5): (C₁-C₄)-Alkyl;
- R(6): (C₁-C₄)-Alkyl;

bedeuten und die Reste R(3) und X wie oben definiert sind, in allen ihren stereoisomeren Formen und Mischungen davon, und ihre physiologisch verträglichen Salze.

Ganz besonders bevorzugt sind Verbindungen der Formel (I), in denen
- R(1): 1. (C₁-C₃)-Alkyl, unsubstituiert oder substituiert mit einem Rest aus der Reihe Fluor, Methoxy, Ethoxy, CO-O-(C₁-C₃)-Alkyl und CO₂H;
2. (C₁-C₃)-Alkyl-Cyclohexyl-,
3. Phenyl, substituiert oder unsubstituiert mit einem Rest aus der Reihe Fluor und Methoxy;
4. (C₁-C₃)-Alkyl-Phenyl, wobei der Phenylrest unsubstituiert oder substituiert ist mit einem Rest aus der Reihe Fluor und Methoxy;
5. Furanyl, Thienyl oder Pyridyl;
- R(2): 1. Wasserstoff;
2. (C₁-C₆)-Alkyl, unsubstituiert oder substituiert mit einem Rest aus der Reihe Fluor, Methoxy und Ethoxy;
3. Phenyl, unsubstituiert oder substituiert mit einem Rest aus der Reihe Fluor und Methoxy;
4. Cyclopropyl oder Cyclohexyl;
- R(4): 1. Wasserstoff;
2. Methyl; und
3. Chlor;
- R(5): Propyl und Butyl, insbesondere n-Propyl, iso-Propyl und 2-iso-Butyl;
- R(6): Methyl, Ethyl, n-Propyl, iso-Propyl, n-Butyl und iso-Butyl;

bedeuten und die Reste R(3) und X wie oben definiert sind, in allen ihren stereoisomeren Formen und Mischungen davon, und ihre physiologisch verträglichen Salze.

Ganz besonders bevorzugt sind auch Verbindungen der Formel (II), 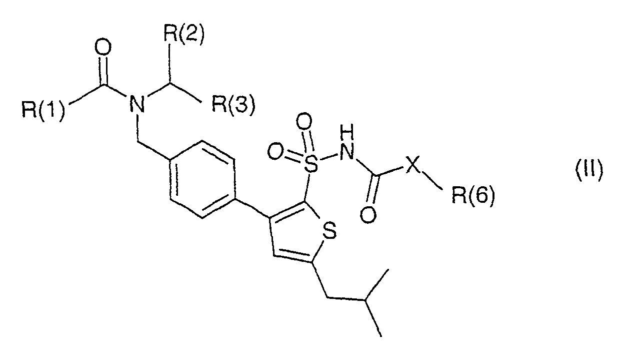
in der die Reste R(1), R(2), R(3), R(6) und X die oben angeführte Bedeutung besitzen, in allen ihren stereoisomeren Formen und Mischungen davon, und ihre physiologisch verträglichen Salze.

Die Erfindung betrifft weiterhin Verfahren zur Herstellung der Verbindungen der Formel (I), die durch die im folgenden wiedergegebenen Reaktionsschritte gekennzeichnet sind:
a) Thiophen-3-boronsäuren der Formel (III), 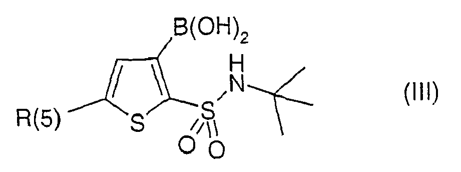
   in der R(5) die oben angeführte Bedeutung besitzt und deren Herstellung aus EP-A 512 675 bekannt ist, werden mit p-Brom-benzaldehyden der Formel (IV), 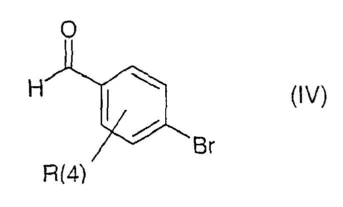
   in denen R(4) wie oben definiert ist, zu Verbindungen der Formel (V), 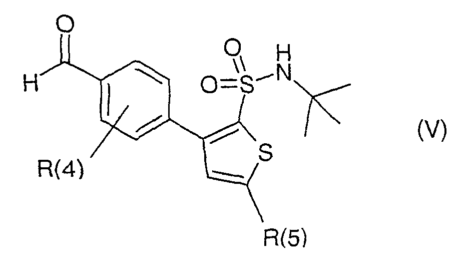
   in denen R(4) und (R5) die oben angeführte Bedeutung besitzen, umgesetzt. Diese Suzuki-Typ Cross-coupling-Reaktion erfolgt vorzugsweise unter Verwendung von Palladium(II)acetat und Triphenylphosphin oder Tetrakistriphenylphosphinpalladium als Katalysatoren in Gegenwart einer Base wie z.B. Cäsium- oder Kaliumcarbonat beispielsweise in Lösungsmittelgemischen aus Ethanol und Toluol bei Temperaturen bis zum Siedepunkt der Lösungsmittel; entsprechende Reaktionen werden in zum Beispiel in Synthetic Commun. 11 (1981) 513, J. Med. Chem. 38 (1995) 2357-2377 und Liebigs Ann. 1995, 1253-1257 beschrieben.
b) Die Verbindungen der Formel (V) lassen sich mit primären Amino-Verbindungen der Formel (VI), 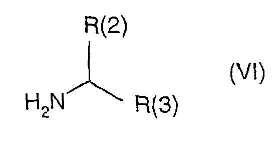
   in denen R(2) und R(3) wie oben definiert sind, zu Verbindungen der Formel (VII), 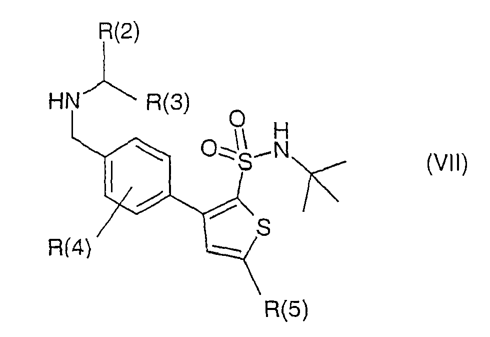
   in denen die Reste R(2), R(3), R(4) und R(5) die oben angeführte Bedeutung besitzen, überführen. Diese reduktive Aminierung erfolgt vorzugsweise durch Umsatz der Amine der Formel (VI) mit den Aldehyden der Formel (V) in einem inerten Lösungsmittel wie z.B. THF in Gegenwart eines Reduktionsmittels wie Natrium-cyanoborhydrid und Molekularsieb als wasserentziehendes Mittel vorzugsweise bei Raumtemperatur oder auch bei Temperaturen bis zum Siedepunkt des verwandten Lösungsmittels; entsprechende Reaktionen werden z.B. in Synthesis 1975, 135ff beschrieben. Neben NaCNBH₃ können für diese Aminierung alternativ z.B. auch Lithiumaluminiumhydrid LiAlH₄, Natriumborhydrid NaBH₄, Natriumtriacetoxyborhydrid NaBH(OAC)₃ oder H₂, Pd/C als Reduktionsmittel verwendet werden; entsprechende Reaktionen sind z.B. in Tetrahedron Lett. 1987, 28, 749ff, Synthesis 1996, 11, 1325-1330 und Tetrahedron Lett. 1982, 23, 1929ff beschrieben.
c) Durch Acylierung der Verbindungen der Formel (VII) mit Acylchloriden des Typs R(1)-COCl, worin R(1) wie oben definiert ist, resultieren Amide der Formel (VIII), 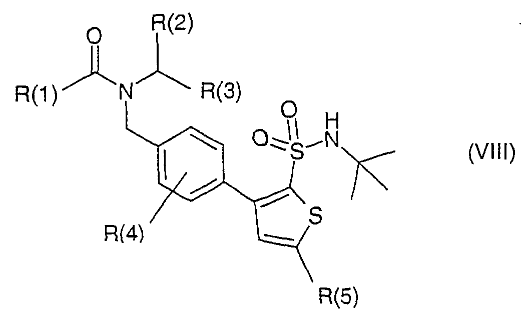
   in denen die Reste R(1), R(2), R(3), R(4) und R(5) die oben angeführte Bedeutung haben. Diese Acylierung erfolgt nach an sich bekannten Methoden durch Umsatz der Verbindungen der Formel (VII) mit käuflich erhältlichen oder durch Behandlung mit Thionylchlorid aus den entsprechenden Carbonsäuren zugänglichen Carbonsäurechloriden in einem auf Rückfluß erhitzten inerten organischen Lösungsmittel wie z.B. CH₂Cl₂ in Gegenwart einer organischen oder anorganischen Base.
d) Die Verbindungen der Formel (VIII) werden durch Abspaltung der *tert*.-Butyl-Schutzgruppe in die Sulfonamide der Formel (IX). 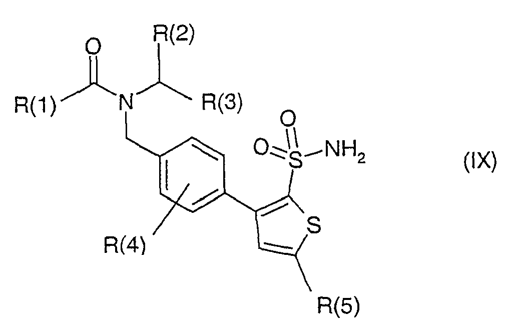
   in denen R(1), R(2), R(3), R(4) und R(5) wie oben definiert sind, überführt. Diese Abspaltung erfolgt bevorzugt durch Behandlung der Verbindungen der Formel (VIII) mit organischen Säuren wie beispielsweise konzentrierter Trifluoressigsäure in Gegenwart von Anisol.
e) Aus den Sulfonamiden der Formel (IX) können durch Umsatz mit R(6)-substituierten Chlorameisensäureestern, worin R(6) wie oben beschrieben ist, in die Sulfonylurethane der Formel (Ia), 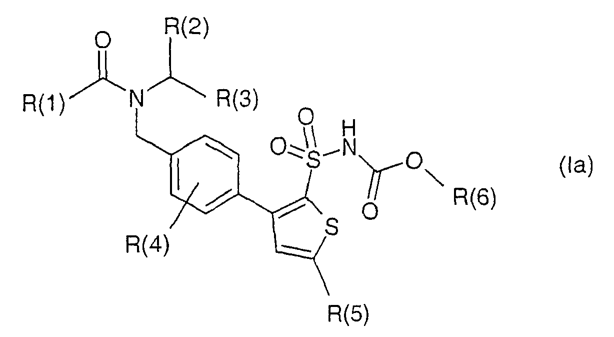
   in der R(1), R(2), R(3), R(4), R(6) wie oben definiert sind, hergestellt werden. Diese Umsetzung erfolgt dabei in Gegenwart einer Base wie beispielweise Pyridin und eines Acylierungsbeschleunigers wie 4-Pyrrolidino-pyridine bei Temperaturen von RT bis 150°C, vorzugsweise jedoch bei RT.
f) Aus den Sulfonamiden der Formel (IX) können durch Behandlung mit R(6)-substituierten Isocyanaten, worin R(6) wie oben beschrieben ist, die Sulfonylharnstoffe der Formel (Ib), 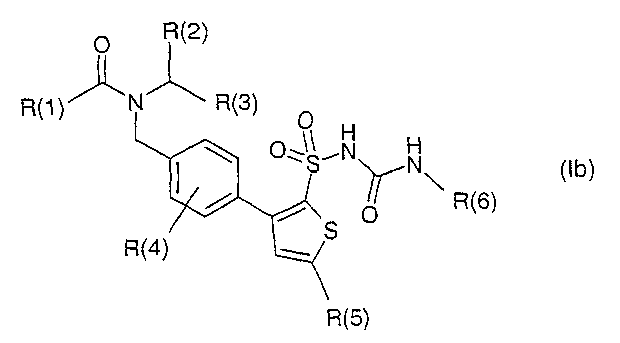
   in der (R1), R(2), R(3), R(4) und R(6) wie oben definiert sind, gewonnen werden. Die Umsetzung mit den R(6)-substituierten Isocyanaten erfolgt dabei in Gegenwart einer Base in einem inerten Lösungsmittel bei Temperaturen von RT bis 150°C.

Als Basen eignen sich z.B. Alkalimetall- oder Erdalkalimetallhydroxide, -hydride,-amide oder -alkoholate, wie Natriumhydroxid, Kaliumhydroxid, Calciumhydroxid, Natriumhydrid, Kaliumhydrid, Calciumhydrid, Natriumamid, Kaliumamid, Natriummethylat, Natriumethylat oder Kalium-*tert*.-butylat. Als inerte Lösungsmittel eignen sich Ether wie THF, Dioxan, Ethylenglykoldimethylether oder Diglyme, Ketone wie Aceton oder Butanon, Nitrile wie Acetonitril, Nitroverbindungen wie Nitromethan, Ester wie Ethylacetat, Amide wie DMF oder N-Methylpyrrolidon, Hexamethylphosphorsäuretriamid, Sulfoxide wie DMSO und Kohlenwasserstoffe wie Benzol, Toluol oder Xylole. Weiterhin eignen sich auch Gemische dieser Lösungsmittel untereinander.
Die Sulfonylharnstoffe der Formel (Ib) sind auch durch Umsetzung von Aminen R(6)-NH₂ mit Sulfonylisocyanat-Derivaten, die aus den Sulfonamiden der Formel (IX) beispielsweise durch Behandlung mit Phosgen oder einem Phosgenersatzstoff wie Triphosgen resultieren, nach dem Fachmann bekannten Methoden herstellbar.
Die Sulfonylharnstoffe der Formel (Ib) lassen sich alternativ auch durch Umsetzung der Sulfonamide der Formel (IX) mit 2,2,2-Trichloracetamid-Derivaten eines geeigneten Amins R(6)-NH₂ in Gegenwart einer Base in einem inerten, hochsiedenden Lösungsmittel wie z.B. DMSO oder aus den durch Umsatz mit Chlorameisäureethylester zugänglichem entsprechenden Sulfonylurethan der Formel (la) durch Einwirkung des entsprechenden Amins R(6)-NH₂ in einem inerten, hochsiedenden Lösungsmittel wie z.B. Toluol bei Temperaturen bis zum Siedepunkt des jeweiligen Lösungsmittels darstellen, welches beispielsweise in J. Med. Chem. 38 (1995) 2357-2377 und in Bioorg. Med. Chem. 5 (1997) 673-678 beschrieben ist.
Die Herstellung der N-unsubstituierten Sulfonylharnstoffe der Formel (Ib), in denen R(6) für Wasserstoff steht, erfolgt durch Verseifung der nach Umsatz der Sulfonamide der Formel (IX) mit Bromcyan in der Gegenwart von K₂CO₃ in Acetontril resultierenden Sulfonamidonitrile mit Schwefelsäure, vorzugsweise bei Temperaturen von -10 - 0 °C.

Nach an sich bekannten Methoden, wie sie in der Literatur (z.B. in den Standardwerken wie Houben-Weyl, Methoden der Organischen Chemie, Georg Thieme Verlag, Stuttgart, Organic Reactions, John Wiley & Sons, Inc., New York oder Larock, Comprehensive Organic Transformations, VCH, Weinheim) können durch alkalische Hydrolyse der Estergruppierungen in den Verbindungen der Formel (I) dann die entsprechenden Carbonsäuren der Formel (I) hergestellt werden.

Das vaskuläre Endothelium ist ein metabolisch aktives Organ mit einer Vielzahl regulatorischer Funktionen, daß zur Synthese und Freisetzung vasoaktiver Substanzen befähigt ist. Eine Dysfunktion der gefäßauskleidenden Endothelschicht wird mit der Pathogenese verschiedener kardiovaskulärer Erkrankung wie Artheriosklerose und Hypertension korreliert (Eur. J. Clin. Invest. 1993, 23, 670-685). Eine endotheliale Dysfunktion ist durch eine reduzierte Synthese und/oder Freisetzung der vasorelaxierenden, vasoprotektiven, antithrombotisch und antiproliferative wirksamen Botenstoffe NO und cGMP charakterisiert, die eine wesentliche Rolle in der Prevention und Regression des vaskulären Remodelling und der arteriellen Hypertension spielen. Substanzen, die in der Lage sind, die Synthese und Freisetzung dieser Botenstoffe zu stimulieren, sind daher potentiell wertvolle Arzneimittel zur Behandlung aller Krankheiten, die durch eine endotheliale Dysfunktion gekennzeichnet sind.

Durch eine Vielzahl von publizierten Experimenten wurde belegt, daß ein Abbauprodukt des Renin-Angiotensin-Systems, das Heptapeptid Angiotensin-(1-7), ein potentes, endogenes Effektorhormon des Renin-Angiotensin-Systems ist (Hypertension 1991, 18 [Suppl III]: III-126-II133), dessen biologische Wirkung durch die Stimulation spezifischer Rezeptoren, die bevorzugt Angiotensin-(1-7) binden, hervorgerufen wird (Peptides 1993, 14, 679-684, Hypertension 1997, 29 [part 2]: 388-393)). Diese Wirkung ist in vielen Fällen der des vasokonstriktorischen Hormons Angiotensin II entgegengerichtet bzw. steht dieser gegenregulatorisch gegenüber (Hypertension 1997, 30 [part 2]: 535-541, Regulatory Peptides 1998, 78, 13-18).
In Hypertension 1992, 19 [suppl. II]: II-49-II-55 und in Am. J. Cardiol. 1998, 82, 17S-19S wurde aufgezeigt, daß Angiotensin-(1-7) die Produktion und/oder die Freisetzung von NO/cGMP und der Prostaglandine E₂ und I₂ stimuliert, welches durch Vorbehandlung mit AT₁- und AT₂-Rezeptor-Antagonisten nicht blockiert wird.
In Hypertension 1996, 27 [part 2]: 523-528 wurde eine endothelabhängige Relaxation an intakten Koronararterien von Hunden und Schweinen sowie in J. Cardiovasc. Pharmacol. 1997, 30, 676-682 eine endothelabhängige Relaxation intakter, durch KCl vorkontrahierter Rattenaorten durch Angiotensin-(1-7) beschrieben, die durch AT₁₋Rezeptor-Antagonisten nicht beeinflußt wird.

In Peptides 1993, 14, 679-684 und in Am. J. Physiol. 1995, 269: H313-H319 wurde die blutdrucksenkende Wirkung von Angiotensin-(1-7) bei Dauerinfusion über eine osmotische Minipumpe in spontan hypertensiven Ratten aufgezeigt, wobei Angiotensin-(1-7) in normotensiven Ratten in gleicher Dosis keine Wirkung auf den Blutdruck hatte. Komplementär zu diesen Untersuchungen wurde in Hypertension 1998, 31: 699-705 demonstriert, daß die Infusion eines Angiotensin-(1-7)-Antikörpers dem mittleren arteriellen Blutdruck in wachen, spontan hypertensiven Ratten, die mit Lisinopril und Losartan vorbehandelt waren, erhöht.
In Am. J. Hypertension 1998, 11: 137-146 wurde demonstriert, daß in Menschen mit essentieller Hypertonie deutlich niedrigere Plasmaspiegel von Angiotensin-(1-7) nachweisbar sind als in normotensiven Menschen.
In Hypertension 1996, 28, 104-108 wurde die anti-proliferative Wirkung von Angiotensin-(1-7) auf vaskuläre Glattmuskelzellen und in Hypertension 1999, 33 [part II]: 207-211 die Hemmung der Proliferation von Glattmuskelzellen nach vaskulärer Gewebsschädigung belegt.
Darüberhinaus zeigte Angiotensin-(1-7) in Kochsalz-beladenen, anästhesierten normotensiven Wistar-Ratten auch renale Effekte wie eine erhöhte Natriurese und Diurese (Am. J. Physiol. 1996, 270, F141-F147).

Die hier beschriebenen Verbindungen der Formel (I) sind potente, nicht-peptidische Agonisten der postulierten endothelialer Angiotensin-(1-7)-Rezeptoren. Sie mimikriieren daher die vorstehend beschriebene, dem Angiotensin II entgegengerichtete biologische Wirkung des Peptidhormons Angiotensin-(1-7), die auf die Produktion und/oder Freisetzung von cGMP und NO aus dem Endothel zurückzuführen ist, ohne dabei dem raschen metabolischen Abbau dieses Hormons zu unterliegen. Die beschriebenen Verbindungen der Formel (i) eignen sich daher allgemein zur Therapie und/oder Prävention von Krankheiten, die primär oder sekundär durch eine reduzierte Produktion und/oder Freisetzung der vasorelaxierenden, antithrombotischen und kardioprotektiven Botenstoffe cyclisches 3',5'-Guanosinmonophoshat (cGMP) und Stickstoffmonoxid (NO) verursacht oder zumindest mitverursacht werden. Durch die Stimulation der Produktion und/oder Freisetzung dieser vasorelaxierenden, antithrombotischen und kardioprotektiven Botenstoffe sind die beschriebenen Angiotensin-(1-7)-Rezeptor-Agonisten der Formel (I) insbesondere potentiell wertvolle Arzneimittel zur Behandlung und Prävention von Bluthochdruck, Herzhypertrophie, Herzinsuffizienz, koronaren Herzerkrankungen wie der Angina Pectoris und einer endothelialen Dysfunktion bzw. endothelialer Schädigungen, zum Beispiel als Folge atheriosklerotischer Prozesse oder beim Diabetis mellitus.

Die Stimulation endothelialer Angiotensin-(1-7)-Rezeptoren durch die Agonisten der Formel (I) verursacht die Freisetzung vasodilatorischer und organprotektiver Autacoide. Dieser Mechanismus unterscheidet sich dabei vom dem der ACE-Hemmung und AT₁-Rezeptor-Blockade durch die Vermeidung entweder von erniedrigtem Gewebs-Angiotensin II (bei ACE-Hemmern) oder von zur Zeit noch nicht abzuschätzender Effekten, die mit erhöhten ANG II-Plasmawerten (bei AT₁-Rezeptor-Antagonisten) verbunden sind.

Die Verbindungen der Formel (I) und ihre physiologisch verträglichen Salze können somit am Tier, bevorzugt am Säugetier, und insbesondere am Menschen als Arzneimittel für sich allein, in Mischungen untereinander oder zusammen mit anderen Wirkstoffen verwendet werden, insbesondere in Form von pharmazeutischen Präparaten. Gegenstand der vorliegenden Erfindung ist daher die Verwendung von Verbindungen der Formel (I) und/oder ihren physiologisch verträglichen Salzen zur Herstellung eines Medikaments zur Therapie oder Prävention der vorstehend genannten Krankheitsbilder, sowie pharmazeutische Präparate, die eine wirksame Dosis mindestens einer Verbindung der Formel (I) und/oder eines physiologisch verträglichen Salzes davon als aktiven Bestandteil neben üblichen, pharmazeutisch einwandfreien Trägerstoffen und/oder Hilfsstoffen enthalten. Die pharmazeutische Zubereitungen können für eine enterale oder eine parenterale Anwendung bestimmt sein und enthalten normalerweise 0.5 bis 90 Gewichtsprozent der Verbindung der Formel (I) und/oder ihrer physiologisch verträglichen Salze. Die Menge an Wirkstoff der Formel (I) und/oder dessen physiologisch verträglichen Salzen in den pharmazeutischen Präparaten beträgt im allgemeinen 0.2 bis 500 mg, vorzugsweise 1 bis 300 mg.

Erfindungsgemäß einsetzbare Arzneimittel, die die Verbindungen der Formel (I) und/oder ihre physiologisch verträglichen Salze enthalten, können enteral, zum Beispiel oral oder rektal, verabreicht werden, zum Beispiel in Form von Pillen, Tabletten, Filmtabletten, Dragees, Granulaten, Hart- und Weichgelatinekapseln, Lösungen wie wäßrigen, alkoholischen oder öligen Lösungen, Säften, Tropfen, Sirupen, Emulsionen oder Suspensionen. Die Verabreichung kann auch parenteral erfolgen, zum Beispiel subkutan, intramuskulär oder intravenös in Form von Injektionslösungen oder Infusionslösungen. Weitere in Betracht kommende Applikationsformen sind zum Beispiel die perkutane oder topische Applikation, zum Beispiel in Form von Salben, Cremes, Pasten, Lotionen, Gelen, Sprays, Puder, Schäumen, Aerosolen oder Lösungen, oder die Verwendung in Form von lmplantaten.

Die Herstellung der erfindungsgemäß einsetzbaren pharmazeutischen Präparate kann nach den bekannten Standardverfahren zur Herstellung pharmazeutischer Präparate erfolgen. Dazu werden ein oder mehrere Verbindungen der Formel (I) und/oder ihre physiologisch verträglichen Salze zusammen mit einem oder mehreren festen oder flüssigen galenischen Trägerstoffen und/oder Zusatzstoffen oder Hilfsstoffen und, wenn gewünscht, in Kombination mit anderen Arzneimittel-wirkstoffen mit therapeutischer oder prophylaktischer Wirkung, zum Beispiel Herz-Kreislauf-aktiven Arzneimitteln wie etwa Calcium-Antagonisten, ACE-Hemmern, AT1-Rezeptor-Antagonisten, NO-Donoren, Endothelin-Rezeptor-Antagonisten, K-Kanal-Öffnern, Phosphodiesterase-Hemmern, Diuretika oder α- und β-Blockern, in eine geeignete Verabreichungsform bzw. Dosierungsform gebracht, die dann als Arzneimittel in der Humanmedizin oder Veterinärmedizin verwendet werden kann.

Als Trägerstoffe kommen organische oder anorganische Substanzen in Frage, die sich für die enterale (zum Beispiel orale) oder parenterale (zum Beispiel intravenöse) Applikation oder topische Anwendungen eignen und mit den Wirkstoffen der Formel (I) nicht reagieren, beispielsweise Wasser, pflanzliche Öle, Alkohole wie Ethanol, Isopropanol oder Benzylalkohole, 1,2-Propandiol, Polyethylenglykole, Glycerintricacetat, Gelatine, Kohlenhydrate wie Lactose oder Stärke, Magnesium-stearat, Talk, Lanolin, Vaseline, Acetonitril, Dimethylformamid, Dimethylacetamid. Zur oralen und rektalen Anwendung dienen insbesondere Arzneiformen wie Tabletten, Dragees, Kapseln, Lösungen, vorzugsweise ölige oder wäßrige Lösungen, Sirupe, Säfte oder Tropfen, ferner Suspensionen oder Emulsionen. Es können auch Gemische von zwei oder mehreren Trägerstoffen eingesetzt werden, zum Beispiel Gemische von zwei oder mehr Lösungsmitteln, insbesondere auch Gemische von einem oder mehreren organischen Lösungsmitteln mit Wasser. Als Zusatzstoffe oder Hilfsstoffe können die pharmazeutischen Präparate zum Beispiel Stabilisierungs- und/oder Netzmittel, Emulgatoren, Salze zum Beispiel zur Beeinflussung des osmotischen Druckes, Gleit-, Konservierungs-, Farb- und Geschmacks- und/ oder Aromastoffe, Puffersubstanzen enthalten. Sie können falls erwünscht auch einen oder mehrerer weitere Wirkstoffe enthalten, zum Beispiel ein oder mehrere Vitamine. Die Verbindungen der Formel (I) und/deren physiologisch verträgliche Salze können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht.

Die Dosierung des zu verabreichenden Wirkstoffs der Formel (I) und/oder eines physiologisch verträglichen Salze davon bei der erfindungsgemäßen Verwendung hängt vom Einzelfall ab und ist wie üblich für eine optimale Wirkung den individuellen Gegebenheiten anzupassen. So hängt sie ab von der Art und Stärke der zu behandelnden Krankheit sowie von Geschlecht, Alter, Gewicht und individueller Ansprechbarkeit des zu behandelnden Menschen oder Tieres, von der Wirkstärke und Wirkdauer der eingesetzten Verbindungen, davon, ob akut oder chronisch therapiert wird oder Prophylaxe betrieben wird, oder davon, ob neben Verbindungen der Formel (I) weitere Wirkstoffe verabreicht werden. Im allgemeinen ist ein Dosisbereich zur Behandlung der vorgenannten Krankheitsbilder im Menschen von etwa 0.1 mg bis etwa 100 mg pro kg und Tag bei Verabreichung an einen ca. 75 kg schweren Erwachsenen zur Erzielung der angestrebten Wirkung angemessen. Bevorzugt ist ein Dosisbereich von 1 bis 20 mg pro kg und Tag (jeweils mg pro kg Körpergewicht). Die Tagesdosis kann dabei als Einzeldosis verabreicht werden oder in mehrere, zum Beispiel ein, zwei, drei oder vier, Einzeldosen aufgeteilt werden. Sie kann auch kontinuierlich verabreicht werden. Gegebenenfalls kann es, je nach individuellem Verhalten, erforderlich werden, von der angegebenen Tagesdosis nach oben oder nach unten abzuweichen. Pharmazeutische Präparate enthalten normalerweise 0.2 bis 500 mg, vorzugsweise 1 bis 300 mg Wirkstoff der Formel (I) und/oder dessen physiologisch verträgliche Salze.

In den folgenden Assays (Test 1 und 2) wurde die Affinität der Verbindungen der Formel (I) zu Angiotensin-(1-7)-Bindungsstellen sowie deren agonistischen Eigenschaften an Endothelzellen nachgewiesen:

### Test 1: Bindungsassay:

Die Messung der Affinität der Verbindungen der Formel (I) zu Angiotensin-(1-7)-Rezeptoren erfolgte durch Ligandenverdrängungsexperimenten an Membranpräparationen von primären Rinderaorten-Endothelzellen, wie sie beispielsweise auch in Hypertension, 1997; 29[part 2]:388-393 beschrieben sind.

### a) Membranpräparation:

Nach Gewinnung von Endothelzellen von Rinderaorten (Test 1, a)), wurden die Zellen bis zum Erreichen ihrer Konfluenz in 75 cm² Kulturflaschen (Becton Dickinson, Heidelberg) kultiviert. Danach wurden die Zellen mit eiskaltem Phosphat-NaCl-EDTA-Puffer (50 mmol/L NaHPO₄, 0.15 mol/L NaCl, 5 mmol/L EDTA, pH 7.2) aufgenommen, mit einem Gummischaber abgelöst und zentrifugiert (1500 x g, 5 Min). Das resultierende Zellpellet wurde zur späteren Membranpräparation eingefroren (-80°C). Das aufgetaute Zellpellet wurde in eiskaltem Phosphat-NaCl-EDTA-Puffer homogenisiert (Glass/Teflon Potter, 1000 U/min, 10 strokes). Die Membranisolierung erfolgte durch anschließende Zentrifugation (30 000 x g, 20 Min.) des Zellhomogenates. Das so gewonnene Zellpellet wurde in modifiziertem HEPES-Puffer (10 nmol/L HEPES, 0.1 mol/L NaCl, 5 mmol/L MgCl₂, pH 7.4) mit Zusatz von 0.2% Rinderserumalbumin und einem Proteasen-Inhibitoren-Cocktail (Complete™, Boehringer Mannheim) resuspendiert. Nach anschließender Proteinbestimmung (nach Lowry) der Membransuspension wurde diese sofort für den Ligandenbindungsversuch verwendet.

### b) Bindungsexperimente:

Die Versuche wurden auf 96-well Opak-Platten, die mit Durapore-Filtern (0.65 µm Porengröße; Millipore, Eschborn) bestückt sind. Vor Beginn des Versuches wurden die Filter mit 1% Rinderserumalbumin für 30 Min. vorbehandelt, um die unspezifische Bindung des radioaktiven Liganden und der kalten Substanzen an das Filtermaterial zu minimieren. Die Inkubation erfolgte in einem Gesamtvolumen von 200 µl: 50 µl ¹²⁵ J-ANG-(1-7), 20 µl kaltes, nicht-radioaktives ANG-(1-7) oder Testsubstanzen der Formel (I), 30 µl Puffer und 100 µl Membranen (20 µg Protein). Die Bindungsreaktion wurde durch Zugabe des radioaktiven Liganden gestartet. Die Inkubation der Proben erfolgte unter ständigem Schütteln bei Zimmertemperatur für 45 Min. Die Bindungsreaktion wurde mittels Vakuumfiltration (-20 kPa Vakuum; Multiscreen Filtrationssystem, Millipor, Eschborn) beendet. Um die nicht-membrangebundene, freie Radioaktivität vollständig zu entfernen, wurden die Filter 2mal mit 250 µl eiskaltem Phosphat-NaCl-EDTA-Puffer (50 mmol/L NaHPO₄, 0.15 mol/L NaCl, 5 mmol/L EDTA, pH 7.2) unter Vakuum gewaschen und dann getrocknet. Der radioaktive Gehalt auf den getrockneten Filtern wurde mittels eines Gammmazählers bestimmt.
Für die Kompetitionsversuche (Bestimmung von "Einzelwerten" oder IC₅₀-Werten) wurde eine Konzentration von 7.5 bis 10 nmol/L ¹²⁵ J-ANG-(1-7) (spezifische Aktivität 1500-2100 mCi/mg) eingesetzt, mit und ohne aufsteigenden Konzentrationen der Testsubstanzen der Formel (I). Die unspezifische Bindung wurde jeweils in Gegenwart von 10 µmol/L nicht-radioaktivem ANG-(1-7) gemessen.

### c) Ergebnisse:

| Beispiel | IC₅₀ |
|---|---|
| 4 | 21 nM |
| 9 | 30 nM |

Diese für die Verbindungen aus den Beispielen 4 und 9 exemplarisch aufgeführte Wert belegt die hohe Affinität der Verbindungen der Formel (I) zum Angiotensin-(1-7)-Rezeptoren an Endothelzellen.

Bezüglich ANGII-Rezeptoren des AT₁- und des AT₂-Typs weisen die Verbindungen der Formel (I) dabei keine bzw. nur eine vernachlässigbare (> 10⁻⁶ M) Affinität auf.

### Test 2: Funktionaler Assay:

Die Messung der stimulierenden Wirkung der Verbindungen der Formel (I) auf die Produktion von intrazellulärem cGMP als Marker für die Produktion und Freisetzung von NO in Endothelzellen erfolgte an primär kultivierten Endothelzellen von Rinderaorten, wie sie beispielweise in J. Pharmacol. Exp. Ther. 1992, 262, 729-733 beschrieben ist.

### a) Zellkulturen:

Nach enzymatischer Digestion (Dispase II; Boehringer, Mannheim) der Endothelzellen von der Rinderaorta wurden die Endothelzellen in Kulturmedium (Dulbecco's modifiziertes Eagle's Ham's F 12 Medium 1:1 mit Penicillin (10 U/L), Streptomycin (10 ug/L), L-Glutamin (1 mmol/L), Glutathion and L-(+)-Ascorbinsäure (jeweils 5 mg/L) und hitze-inaktiviertes fetales Kälberserum (20%)) aufgenommen, 1 mal gewaschen (Zentrifugation bei 170 x g, 10 min) und in Kulturmedium resuspendiert. Die so gewonnene Zellsuspension wurde in 6-well Platten (Nunc Intermed, Wiesbaden) ausgesät (-250 µg Protein oder 3 x 10⁻⁵ Zellen per well), mit Kulturmedium aufgefüllt und bei 37°C in einem befeuchteten, und mit 95%O₂-5%CO₂ begasten Inkubator gehalten.

### b) cGMP-Bestimmungen:

Nach Erreichen der Konfluenz (6-8 Tage nach der Aussaat) wurde das Kulturmedium entfernt und der Zellmonolayer 2mal mit warmer HEPES/Tyrode's Lösung gewaschen. Danach wurden die Zellen für 15 Min. bei 37°C in HEPES/Tyrode's Lösung, die IBMX (3-Isobutyl-1-methylxanthin, 10⁻⁴ mol/L, Serva, Heidelberg) enthält, vorinkubiert. Die Inkubation wurde durch Zugabe von SOD (Superoxiddismutase von Rindererythrozyten, 3 x 10⁻⁷ mol/L, Serva, Heidelberg) und den Testsubstanzen der Formel (I) in den angegeben Konzentrationen gestartet. Nach der entsprechenden Inkubationszeit wurde das Inkubationsmedium abgsaugt, die zurückbleibenden Zellen sofort in 1 N Ameisensäure-Aceton (v/v,15:85) extrahiert und abgeschabt. Die erhaltene Suspension wurde ultrabeschallt (10 Sek.) und dann abzentrifugiert (3000 x g, 10 Min). Für die Bestimmung von cGMP mittels Radioimmunoassay (New England Nuclear, Bosten, MA) wurde der Überstand lyophylisiert und in Natrium-Acetat-Puffer (0.05 mol/L;pH 6.2) aufgenommen. Der Gehalt (pmol) an intrazellulärem cGMP wurde auf mg Zellprotein bezogen.

### c) Ergebnisse:

| Beispiel | EC₅₀ |
|---|---|
| 4 | 6.0 x 10⁻⁷ M |
| 9 | 0.4 x 10⁻⁷ M |

Die aufgeführten Werte für die in den Beispielen 4 und 9 beschriebene Verbindungen als repräsentativer Vertreter der beanspruchten Verbindungen belegt die agonistische Wirkung der Verbindungen der Formel (I) auf Angiotensin-(1-7)-Rezeptoren.
Diese Wirkung der Verbindungen aus den Beispielen 4 und 9 auf die Produktion von cGMP als Marker für die NO-Synthese und -Freisetzung wird dabei durch Vorinkubation mit einem Angiotensin II Rezeptor-Antagonisten sowohl des AT₁₋Subtypes wie EXP3174 oder des AT₂-Subtypes wie PD 123,319 nicht beeinflußt. Im Gegensatz dazu wird der beschriebene stimulierende Effekt der Verbindungen aus den Beispielen 4 und 9 auf das cGMP durch Vorinkubation mit einen selektiven Antagonisten der Angiotensin-(1-7)-Rezeptoren, [D-Ala⁷]-Angiotensin-(1-7), der beispielweise in Brain Res. Bull. 1994, 35, 293-298 beschrieben ist, gehemmt, welches die Spezifität dieses funktionalen Effektes belegt.Liste der Abkürzungen:
- abs.: absolut
- cGMP: cyclisches Guanosinmonophoshat
- CH₂Cl₂: Dichlormethan
- DCI: Desorption Chemical lonisation
- DMF: N,N-Dimethylformamid
- EE: Ethylacetat
- ESI: Electron Spray Ionisation
- FAB: Fast Atom Bombardment
- Fp: Schmelzpunkt
- ges.: gesättigt
- h: Stunde(n)
- Min.: Minute(n)
- NO: Stickstoffmonoxid
- RT: Raumtemperatur
- THF: Tetrahydrofuran

Die Erfindung wird durch die nachstehenden Beispiele erläutert, ohne auf diese beschränkt zu sein.

### Beispiele:

### Beispiel 1

### 2-N-Benzoyl-2-N-[4-[2-(n-butyloxycarbonylsulfonamido)-5-isobutyl-3-thienyl]-benzyl]-amino-3-methyl-L-buttersäuremethylester

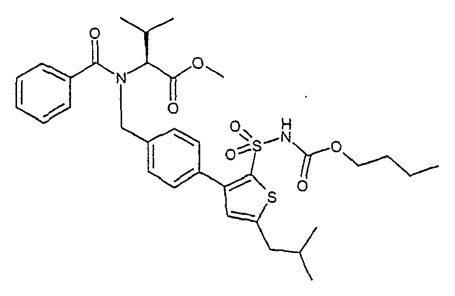

### a) 2-(N-tert.-butyl)-sulfonamido-thiophen

Zu einer Lösung aus 50 g (0.27 mol) 2-Thiophensulfonylchlorid in 500 ml CH₂Cl₂ wurde unter Eiskühlung 85.5 ml (0.82 mol) *N*-*tert*.-Butylamin langsam hinzugetropft und die erhaltene Lösung für 1 h an RT gerührt. Anschließend wurde die Reaktionslösung mit 500 ml 1N Salzsäure versetzt, die organische Phase abgetrennt und diese mit Wasser gewaschen. Nach Trocknung über Na₂SO₄, Abzug des Lösungsmittels und Trocknung im Hochvakuum resultierten schließlich 58.2 g der Titelverbindung in Form eines blaßgelben Öls.
R_{f} (SiO₂, EE/n-Heptan 1:1) = 0.39
MS (ESI): m/z = 220 [M+H]⁺

### b) 5-Isobutyl-2-[(N-tert.-butyl)-sulfonamido]-thiophen

In einer Argon-Atmosphäre wurde zu einer auf -78 °C gekühlte Lösung von 24.2 g (0.11 mol) der Verbindung aus Beispiel 1a) in 450 ml abs. THF 173.2 ml (0.27 mol) einer 15%igen Lösung von *n*-Butyllithium in Hexan getropft und die erhaltene Lösung für 3 h bei -20 °C und für 2 h an RT gerührt. Die Lösung wurde wieder auf -20 °C gekühlt und bei dieser Temperatur mit 15 ml (0.13 mol) 1-Jod-2-methylpropan versetzt. Nach Rühren für 1 h bei 0 °C wurde die Reaktionslösung über Nacht stehengelassen. Anschließend wurden 150 ml ges. Ammoniumchlorid-Lösung hinzugefügt und nach Zusatz von 150 ml Wasser mehrfach mit EE extrahiert. Die vereinten EE-Phasen wurden über Na₂SO₄ getrocknet, eingeengt und der verbliebene Rückstand durch Chromatographie an SiO₂ mit EE/n-Heptan (1:6) als Laufmittel gereinigt, wobei 9.8 g der Titelverbindung in Form eines braunen Öls gewonnen wurden.
R_{f} (SiO₂, EE/n-Heptan 1:4) = 0.28
MS (ESI): m/z = 276 [M+H]⁺

### c) 5-Isobutyl-2-[(N-tert.-butyl)-sulfonamido]-thiophen-3-boronsäure

In einer Argon-Atmosphäre wurde zu einer auf -78 °C gekühlten Lösung aus 9.6 g (35.1 mmol) der Verbindung aus Beispiel 1b) in 350 ml abs. THF langsam 54.4 ml einer 15%igen Lösung von *n*-Butyllithium in Hexan getropft und die erhaltene Lösung unter Rühren innerhalb von 2 h auf RT erwärmt. Nach anschließender Kühlung auf 0 °C wurde mit 8.93 ml (52.2 mmol) Borsäuretrimethylester versetzt und die erhaltene Reaktionslösung für erst für 1 h bei 0 °C und dann 24 h an RT gerührt. Nach Zugabe von 70 ml 2N Salzsäure und 30 Min. Rühren an RT wurde die organische Phase abgetrennt, mit Wasser gewaschen, über Na₂SO₄ getrocknet und das Lösungsmittel im Vakuum entfernt. Chromatographische Reinigung des verbliebenen Rückstandes an SiO₂ mit CH₂Cl₂/Methanol (30:1) als Laufmittel lieferte 10.3 g der Titelverbindung in Form eines hellbraunen Öls.
R_{f} (SiO₂, EE/n-Heptan 1:1) = 0.22
MS (ESI): m/z = 320 [M+H]⁺

### d) 4-[5-Isobutyl-2-[(N-tert.-butyl)-sulfonamido]-3-thienyl-]-benzaldehyd

In einer Argon-Atmosphäre wurde eine Lösung von 3.72 g (14.5 mmol) der Verbindung aus Beispiel 1c) in 75 ml Ethanol langsam zu einer Lösung aus 2.68 g (14.5 mmol) 4-Brombenzaldehyd und 460 mg (0.40 mmol) Tetrakistriphenylphosphinpalladium-(0) in 75 ml Toluol getropft und für 15 Min. an RT gerührt. Nach Zugabe von 16.9 ml einer 2N Cs₂CO₃-Lösung wurde die resultierende Reaktionslösung für 3 h am Rückfluß erhitzt. Anschließend wurde zur Trockene eingeengt, der verbliebene Rückstand in EE aufgenommen, die EE-Lösung mit Wasser gewaschen und nach Trocknung über Na₂SO₄ eingeengt. Chromatographische Reinigung des verbliebenen Rückstandes an SiO₂ mit EE/n-Heptan (1:4) als Laufmittel lieferte 5.46 g der Titelverbindung als schwach gelb gefärbten Feststoff.
Fp: 140-143 °C
R_{f} (SiO₂, EE/n-Heptan 1:2) = 0.47
MS (FAB): m/z = 380 [M+H]⁺

### e) 2-N-[4-[2-(N-tert.-butyl)-sulfonamido-5-isobutyl-3-thienyl]-benzyl]-amino-3-methyl-L-buttersäuremethylester

In einer Argon-Atmosphäre wurde 5.40 g (14.4 mmol) der Verbindung aus Beispiel 1d) in 160 ml abs. THF gelöst und diese Lösung mit 16 g aktiviertem Molekularsieb (5 Angström) und 4.82 g (28.8 mmol) L-Valinmethylester-Hydrochlorid versetzt. Zu dieser Reaktionsmischung wurden nach Rühren für 30 Min. an RT bei 0-5 °C langsam eine Lösung aus 910 mg (14.4 mmol) Natriumcyanoborhydrid in 18 ml Methanol getropft und anschließend über Nacht an RT gerührt. Nach Filtration wurde die erhaltenen Lösung eingeengt und der verbliebene Rückstand durch Chromato-graphie an SiO₂ mit EE/n-Heptan (1:2) als Laufmittel gereinigt. Vereinigung der produkthaltigen Fraktionen und deren Einengung ergab 4.41 g der Titelverbindung in Form eines amorphen Feststoffs.
R_{f} (SiO₂, EE/n-Heptan 1:4) = 0.43
MS (ESI): m/z = 495 [M+H]⁺

### f) 2-N-Benzoyl-2-N-[4-[2-(N-tert.-butyl)-sulfonamido-5-isobutyl-3-thienyl]-benzyl]-amino-3-methy(-L-buttersäuremethylester

In einer Argon-Atmosphäre wurde eine Lösung aus 1.0 g (2.02 mmol) der Verbindung aus Beispiel 1e), 352 µl (3.03 mmol) Benzoylchlorid und 280 µl (2.02 mmol) Triethylamin in 20 ml CH₂Cl₂ für 1 h am Rückfluß erhitzt. Anschließend wurde die Lösung mit Wasser gewaschen, die organische Phase über Na₂SO₄ getrocknet, filtriert und eingeengt. Chromatographische Reinigung des verbliebenen Rückstandes an SiO₂ mit EE/n-Heptan (1:2) als Laufmittel lieferte 1.20 g der Titelverbindung als amorphen Feststoff.
R_{f} (SiO₂, EE/n-Heptan 1:4) =0.12
MS (ESI): m/z = 599 [M+H]⁺

### g) 2-N-Benzoyl-2-N-[4-[2-sulfonamido-5-isobutyl-3-thienyl]-benzyl]-amino-3-methyl-L-buttersäuremethylester

Eine Lösung aus 1.15 g (1.92 mmol) der Verbindung aus Beispiel 1f), 2.35 ml (21.5 mmol) Anisol und 12.2 ml Trifluoressigsäure wurde über 24 h an RT gerührt. Die Reaktionslösung wurde eingeengt und der Rückstand in EE aufgenommen. Die EE-Lösung wurde dann mit Wasser und ges. Kochsalzlösung gewaschen, über Na₂SO₄ getrocknet und eingeengt. Nach chromatographischer Reinigung des erhaltenen Rückstandes an SiO₂ mit EE/n-Heptan (1:2) als Laufmittel resultierten 834 mg der Titelverbindung in Form eines weißen Feststoffs.
Fp: 50 °C (Erweichung)
R_{f} (SiO₂, EE/n-Heptan 1:2) = 0.28
MS (ESI): m/z = 543 [M+H]⁺

### h) 2-N-Benzoyl-2-N-[4-[2-(n-butyloxycarbonylsulfonamido)-5-isobutyl-3-thienyl]-benzyl]-amino-3-methyl-L-buttersäuremethylester

Unter einer Argon-Atmosphäre wurde eine Lösung aus 400 mg (0.74 mmol) der Verbindung aus Beispiel 1g), 13 mg (0.09 mmol) 4-Pyrolidinopyridin und 927 µl (7.37 mmol) Clorameisensäure-*n*-butylester in 6 ml abs. Pyridin über 2 Tage an RT gerührt. Die Reaktionslösung wurde anschließend im Vakuum zur Trockene eingeengt und der Rückstand in CH₂Cl₂ aufgenommen. Die erhaltene Lösung wurde nacheinander mit einer 10%igen Citronensäure-Lösung und mit Wasser gewaschen, über Na₂SO₄ getrocknet und eingeengt. Reinigung des verbliebenen Rückstandes durch Chromatographie an SiO₂ mit CH₂Cl₂/Methanol (20:1) lieferte 470 mg der Titelverbindung in Form eines schwach gelben, amorphen Schaums.
R_{f} (SiO₂, EE/n-Heptan 1:1) = 0.35
MS (ESI): m/z = 643 [M+H]⁺

### Beispiel 2

### 2-N-Benzoyl-2-N-[4-[2-(n-butyloxycarbonylsulfonamido)-5-isobutyl-3-thienyl]-benzyl]-amino-3-methyl-L-buttersäureethylester

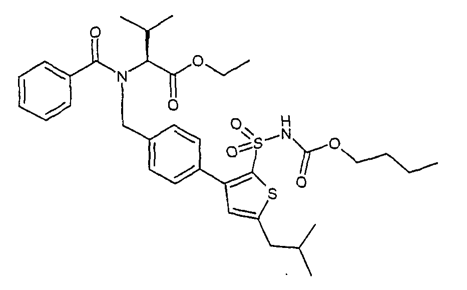

### a) 2-N-(4-[2-(N-tert.-butyl)-sulfonamido-5-isobutyl-3-thienyl]-benzyl]-amino-3-methyl-L-buttersäureethylester

Die Herstellung der Titelverbindung erfolgte durch Umsatz der Verbindung aus Beispiel 1d) mit L-Valinethylester nach dem in Beispiel 1e) angeführten Verfahren. Dabei resultierten aus 600 mg (1.58 mmol) der Verbindung aus Beispiel 1d) und 574 mg (3.16 mmol) L-Valinethylester 430 mg der gewünschten Titelverbindung in Form eines schwach gelbgefärbten Öls.
R_{f} (SiO₂, EE/n-Heptan 1:4) = 0.11
MS (ESI): m/z = 509 [M+H]⁺

### b) 2-N-Benzoyl-2-N-[4-[2-(N-tert.-butyl)-sulfonamido-5-isobutyl-3-thienyl]-benzyl]-amino-3-methyl-L-buttersäureethylester

Die Herstellung der Titelverbindung erfolgte durch Umsatz der Verbindung aus Beispiel 2a) mit Benzoylchlorid nach dem in Beispiel 1f) angeführten Verfahren. Dabei resultierten aus 215 mg (0.42 mmol) der Verbindung aus Beispiel 2a) und 73.7 µl (0.63 mmol) Benzoylchlorid 218 mg der gewünschten Titelverbindung in Form eines weißen, amorphen Schaums.
R_{f} (SiO₂, EE/n-Heptan 1:1) = 0.44
MS (ESI): m/z = 613 [M+H]⁺

### c) 2-N-Benzoyl-2-N [4-[2-sulfonamido-5-isobutyl-3-thienyl]-benzyl]-amino-3-methyl-L-buttersäureethylester

Die Herstellung der Titelverbindung erfolgte durch Behandlung der Verbindung aus Beispiel 2b) mit Trifluoressigsäure nach dem in Beispiel 1g) angeführten Verfahren. Dabei wurden aus 212 mg (0.35 mmol) der Verbindung aus Beispiel 2b) 161 mg der gewünschten Titelverbindung in Form eines weißen, amorphen Schaums gewonnen.
R_{f} (SiO₂, EE/n-Heptan 1:1)= 0.35
MS (ESI): m/z = 557 [M+H]⁺

### d) 2-N-Benzoyl-2-N-[4-[2-(n-butyloxycarbonylsulfonamido)-5-isobutyl-3-thienyl]-benzyl]-amino-3-methyl-L-buttersäureethylester

Die Herstellung der Titelverbindung erfolgte durch Umsatz der Verbindung aus Beispiel 2c) mit Chlorameisensäure-*n*-butylester nach dem in Beispiel 1h) angeführten Verfahren. Dabei resultierten aus 80 mg (0.14 mmol) der Verbindung aus Beispiel 2c) und 180.6 µl (1.44 mmol) Chlorameisensäure-*n*-butylester 69 mg der gewünschten Titelverbindung in Form eines weißen, amorphen Schaums.
R_{f} (SiO₂, EE/n-Heptan 1:1) = 0.29
MS (ESI): m/z = 657 [M+H]⁺

### Beispiel 3

### 2-N-Benzoyl-2-N-[4-[2-(n-butyloxycarbonylsulfonamido)-5-isobutyf-3-thienyl]-benzyl]-amino-3-methyl-L-buttersäure

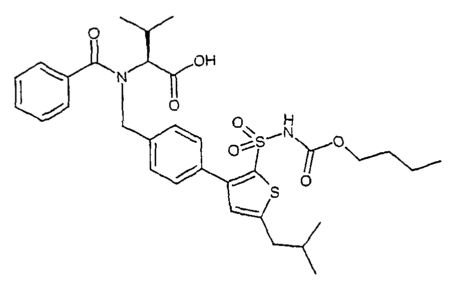

Eine Lösung aus 358 mg (0.56 mmol) der Verbindung aus Beispiel 1h) und 3.7 ml 2N Natronlauge wurde für 3 d an RT gerührt. Anschließend wurde die Reaktions-lösung eingeengt und der pH-Wert der verbliebenen Lösung durch Zugabe von 2 N Salzsäure auf 6 eingestellt. Der ausgefallene Niederschlag wurde abgesaugt, mit wenig Wasser gewaschen und im Hockvakuum getrocknet. Es resultierten 173 mg der Titelverbindung in Form eines weißen, amorphen Feststoffs.
R_{f} (SiO₂, EE/n-Heptan 2:1) = 0.12
MS (FAB): m/z = 629 [M+H]⁺

### Beispiel 4

### 2-N-Benzoyl-2-N-[4-[2-(ethylaminocarbonylsulfonamido)-5-isobutyl-3-thienyl]-benzyl]-amino-3-methyl-L-buttersäuremethylester

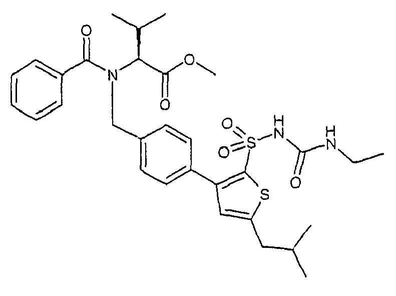

Eine Lösung aus 392 mg (0.72 mmol) der Verbindung aus Beispiel 1g), 228 mg (1.64 mmol) K₂CO₃ und 57.2 µl (0.72 mmol) Ethylisocyanat in 5 ml abs. DMF wurde für 2 h am Rückfluß gerührt. Anschließend wurde die Reaktionslösung mit 26 ml einer 10%igen K₂HPO₄-Lösung versetzt und dann mehrfach mit EE extrahiert. Die EE-Extrakte wurden vereint, über Na₂SO₄ getrocknet und im Vakuum zur Trockene eingeengt. Chromatographische Reinigung des verbliebenen Rückstandes an SiO₂ mit CH₂Cl₂/Methanol (40:1) als Laufmittel lieferte 362 mg der Titelverbindung in Form eines weißen Feststoffs.
Fp: 65 °C (Erweichung)
R_{f} (SiO₂, EE/n-Heptan 1:1)= 0.31
MS (ESI): m/z = 614 [M+H]⁺

### Beispiel 5

### 2-N-Benzoyl-2-N-[4-[2-(ethylaminocarbonylsulfonamido)-5-isobutyl-3-thienyl]-benzyl]-amino-3-methyl-L-buttersäureethylester

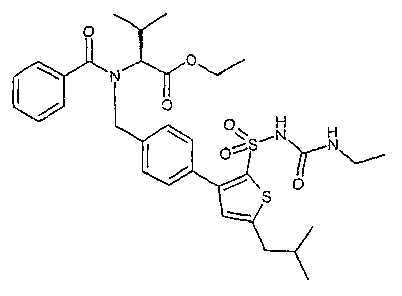

Die Herstellung der Titelverbindung erfolgte durch Umsatz der Verbindung aus Beispiel 2c) mit Ethylisocyanat nach dem in Beispiel 4) angeführten Verfahren. Dabei resultierten aus 80 mg (0.14 mmol) der Verbindung aus Beispiel 2c) und 11.3 µl (0.14 mmol) Ethylisocyanat 89 mg der gewünschten Titelverbindung in Form eines weißen Feststoffs.
Fp: 88-90 °C
R_{f} (SiO₂, EE/n-Heptan 1:1) = 0.12
MS (ESI): m/z = 628 [M+H]⁺

### Beispiel 6

### 2-N-Benzoyl-2-N-[4-[2-(ethylaminocarbonylsulfonamido)-5-isobutyl-3-thienyl]-benzyl]-amino-3-methyl-L-buttersäure

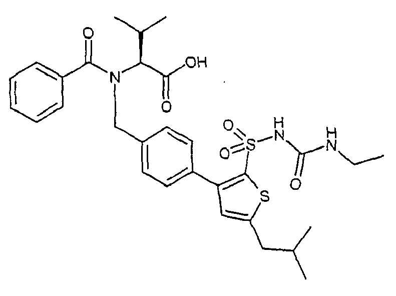

Eine Lösung aus 80 mg (0.13 mmol) der Verbindung aus Beispiel 1h) und 1 ml 6N Natronlauge wurde für 3 d an RT gerührt. Anschließend wurde die Reaktionslösung eingeengt und der pH-Wert der verbliebenen Lösung durch Zugabe von 2 N Salzsäure auf 6 eingestellt. Der ausgefallene Niederschlag wurde abgesaugt, mit wenig Wasser gewaschen und im Hockvakuum getrocknet. Es resultierten 69 mg der Titelverbindung in Form eines weißen Feststoffs.
Fp: 149 °C
R_{f} (SiO₂, EE/n-Heptan 1:2) = 0.12
MS (ESI): m/z = 600 [M+H]⁺

### Beispiel 7

### 2-N-Acetyl-2-N-[4-[2-(n-butyloxycarbonylsulfonamido)-5-isobutyl-3-thienyl]-benzyl]-amino-3-methyl-L-buttersäuremethylester

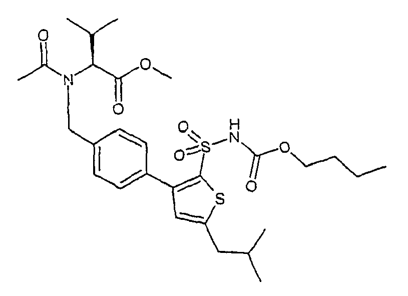

### a) 2-N-Acetyl-2-N-[4-[2-(N-tert.-butyl)-sulfonamido-5-isobutyl-3-thienyl]-benzyl]-amino-3-methyl-L-buttersäuremethylester

Unter einer Argon-Atmosphäre wurde eine Lösung aus 1.0 g (2.02 mmol) der Verbindung aus Beispiel 1e), 280 µl (2.02 mmol) Triethylamin und 216 µl (3.03 mmol) Acetylchlorid in 20 ml CH₂Cl₂ für 3 h am Rückfluß gerührt. Anschließend wurde die Reaktionslösung mit Wasser gewaschen, über Na₂SO₄ getrocknet und im Vakuum zur Trockene eingeengt. Chromatographische Reinigung des verbliebenen Rückstandes an SiO₂ mit EE/*n*-Heptan (1:2) als Laufmittel ergab 854 mg der Titelverbindung in Form eines weißen, amorphen Schaums.
R_{f} (SiO₂, EE/n-Heptan 1:2) = 0.17
MS (ESI): m/z = 537 [M+H]⁺

### b) 2-N-Acetyl-2-N-[4-[2-sulfonamido-5-isobutyl-3-thienyl]-benzyl]-amino-3-methyl-L-buttersäuremethylester

Die Herstellung der Titelverbindung erfolgte durch Behandlung der Verbindung aus Beispiel 7a) mit Trifluoressigsäure nach dem in Beispiel 1g) angeführten Verfahren. Dabei resultierten aus 819 mg (1.53 mmol) der Verbindung aus Beispiel 7a) 510 mg der gewünschten Titelverbindung in Form eines weißen Feststoffs.
Fp: 163-165 °C
R_{f} (SiO₂, EE/n-Heptan 1:2) = 0.10
MS (ESI): m/z = 481 [M+H]⁺

### c) 2-N-Acetyl-2-N-[4-[2-(n-butyloxycarbonylsulfonamido)-5-isobutyl-3-thienyl]-benzyl]-amino-3-methyl-L-buttersäuremethylester

Die Herstellung der Titelverbindung erfolgte durch Umsatz der Verbindung aus Beispiel 7b) mit Chlorameisensäure-*n*-butylester nach dem in Beispiel 1h) angeführten Verfahren. Dabei resultierten aus 240 mg (0.50 mmol) der Verbindung aus Beispiel 7b) und 628 µl (5.00 mmol) Chlorameisensäure-*n*-butylester 270 mg der gewünschten Titelverbindung in Form eines weißen, amorphen Feststoffs.
R_{f} (SiO₂, EE/n-Heptan 1:1) = 0.07
MS (ESI): m/z = 581 [M+H]⁺

### Beispiel 8

### 2-N-Acetyl-2-N-[4-[2-(n-butyfoxycarbonylsuffonamido)-5-isobutyl-3-thienyl]-benzyl]-amino-3-methyl-L-buttersäure

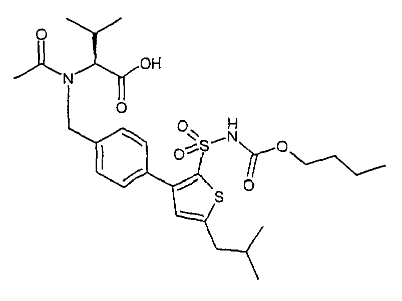

Die Herstellung der Titelverbindung erfolgte durch alkalische Hydrolyse der Verbindung aus Beispiel 7c) durch Behandlung mit Natronlauge nach dem in Beispiel 3 angeführten Verfahren. Dabei resultierten aus 174 mg (0.30 mmol) der Verbindung aus Beispiel 7c) 147 mg der gewünschten Titelverbindung in Form eines weißen, amorphen Feststoffs.
R_{f} (SiO₂, EE/n-Heptan 2:1) = 0.07
MS (FAB): m/z = 567 [M+H]⁺

### Beispiel 9

### 2-N-Acetyl-2-N-[4-[2-(ethylaminocarbonylsulfonamido)-5-isobutyl-3-thienyl]-benzyl]-amino-3-methyl-L-buttersäuremethylester

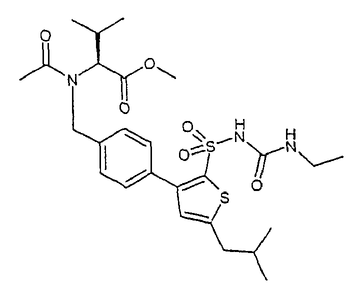

Die Herstellung der Titelverbindung erfolgte durch Umsatz der Verbindung aus Beispiel 7b) mit Ethylisocyanat nach dem in Beispiel 4) angeführten Verfahren. Dabei resultierten aus 234 mg (0.49 mmol) der Verbindung aus Beispiel 7b) 245 mg der Titelverbindung in Form eines weißen Feststoffs.
Fp: 130 °C
R_{f} (SiO₂, EE/n-Heptan 1:1) = 0.11
MS (ESI): m/z = 552 [M+H]⁺

### Beispiel 10

### 2-N-Acetyl-2-N-[4-[2-(ethylaminocarbonylsulfonamido)-5-isobutyl-3-thienyl]-benzyl]-amino-3-methyl-L-buttersäureethylester

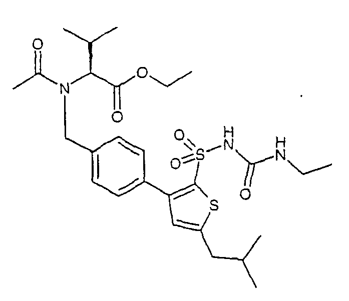

### a) 2-N-Acetyl-2-N-[4-[2-(N-tert.-butyl)-sulfonamido-5-isobutyl-3-thienyl]-benzyl]-amino-3-methyl-L-buttersäureethylester

Die Herstellung der Titelverbindung erfolgte durch Umsatz der Verbindung aus Beispiel 2a) mit Acetylchlorid nach dem in Beispiel 7a) angeführten Verfahren. Dabei resultierten aus 215 mg (0.42 mmol) der Verbindung aus Beispiel 2a) 113 mg der Titelverbindung in Form eines blaßgelben Öls.
R_{f} (SiO₂, EE/*n*-Heptan 1:2) = 0.26
MS (ESI): m/z = 551 [M+H]⁺

### b) 2-N-Acetyl-2-N-[4-[2-sulfonamido-5-isobutyl-3-thienyl]-benzyl]-amino-3-methyl-L-buttersäureethylester

Die Herstellung der Titelverbindung erfolgte durch Behandlung der Verbindung aus Beispiel 10a) mit Trifluoressigsäure nach dem in Beispiel 1g) angeführten Verfahren. Dabei resultierten aus 110 mg (0.20 mmol) der Verbindung aus Beispiel 10a) 74 mg der Titelverbindung in Form eines weißen, amorphen Schaums.
R_{f} (SiO₂, EE/n-Heptan 1:1) = 0.17
MS (ESI): m/z = 495 [M+H]⁺

### c) 2-N-Acetyl-2-N-[4-[2-(ethylaminocarbonylsulfonamido)-5-isobutyl-3-thienyl]-benzyl]-amino-3-methyl-L-buttersäureethylester

Die Herstellung der Titelverbindung erfolgte durch Umsatz der Verbindung aus Beispiel 10b) mit Ethylisocyanat nach dem in Beispiel 4) angeführten Verfahren. Dabei resultierten aus 71 mg (0.14 mmol) der Verbindung aus Beispiel 10b) 52 mg der Titelverbindung in Form eines weißen Feststoffs.
Fp: 81 °C
R_{f} (SiO₂, EE/n-Heptan 1:1) = 0.06
MS (ESI): m/z = 566 [M+H]⁺

### Beispiel 11

### 2-N-Acetyl-2-N-[4-[2-(ethylaminocarbonylsulfonamido)-5-isobutyl-3-thienyl]-benzyl]-amino-3-methyl-L-buttersäure

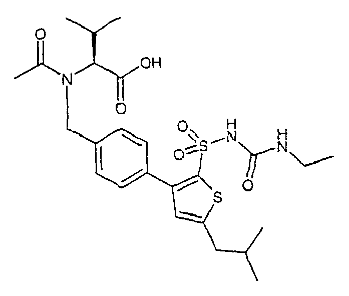

Die Darstellung der Titelverbindung erfolgte durch Behandlung der Verbindung aus Beispiel 9) mit Natronlauge nach dem in Beispiel 6) angeführten Verfahren. Dabei resultierten aus 60 mg (0.11 mmol) der Verbindung aus Beispiel 9) 40 mg der gewünschten Titelverbindung in Form eines weißen Feststoffs.
Fp:136°C
R_{f} (SiO₂, EE/n-Heptan 2:1) = 0.05
MS (ES); m/z = 538 [M+H]⁺

### Beispiel 12

### 2-N-(2-Furanoyl)-2-N-[4-[2-(n-butyloxycarbonylsulfonamido)-5-isobutyl-3-thienyl]-benzyl]-amino-3-methyl-L-buttersäuremethylester

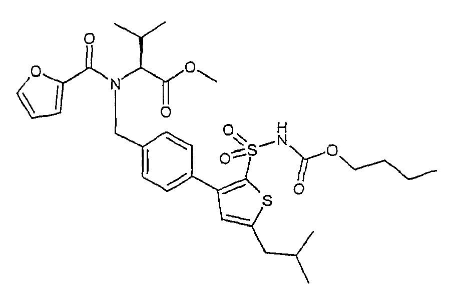

### a) 2-N-(2-Furanoyl)-2-N-[4-[2-(N-tert.-butyl)-sulfonamido-5-isobutyl-3-thienyl]-benzyl]-amino-3-methyl-L- buttersäuremethylester

In einer Argon-Atmosphäre wurde eine Lösung aus 750 mg (1.52 mmol) der Verbindung aus Beispiel 1e), 224 µl (2.27 mmol) Furan-2-carbonsäurechlorid und 210 µl (1.52 mmol) Triethylamin in 18 ml CH₂Cl₂ für 4 h am Rückfluß erhitzt. Anschließend wurde die Reaktionslösung mit Wasser versetzt, die organische Phase abgetrennt, über Na₂SO₄ getrocknet und im Vakuum zur Trockene eingeengt. Reinigung des verbliebenen Rückstandes durch Chromatographie an SiO₂ mit EE/*n*-Heptan (1:3) als Laufmittel lieferte 870 mg der Titelverbindung als weißer Feststoff.
Fp: 59 °C
R_{f} (SiO₂, EE/n-Heptan 1:1) = 0.36
MS (ESI): m/z = 589 [M+H]⁺

### b) 2-N-(2-Furanoyl)-2-N-[4-[2-sulfonamido-5-isobutyl-3-thienyl]-benzyl]-amino-3-methyl-L-buttersäuremethylester

Die Herstellung der Titelverbindung erfolgte durch Behandlung der Verbindung aus Beispiel 12a) mit Trifluoressigsäure nach dem in Beispiel 1g) angeführten Verfahren. Dabei resultierten aus 860 mg (1.47 mmol) der Verbindung aus Beispiel 12a) 487 mg der gewünschten Titelverbindung in Form eines weißen Feststoffs.
Fp: 57 °C
R_{f} (SiO₂, EE/n-Heptan 2:1) = 0.28
MS (ESI): m/z = 533 [M+H]⁺

### c) 2-N-(2-Furanoyl)-2-N-[4-[2-(n-butyloxycarbonylsulfonamido)-5-isobutyl-3-thienyl]-benzyl]-amino-3-methyl-L-buttersäuremethylester

Die Herstellung der Titelverbindung erfolgte durch Umsatz der Verbindung aus Beispiel 12b) mit Chlorameisensäure-*n*-butylester nach dem in Beispiel 1h) angeführten Verfahren. Dabei resultierten aus 227 mg (0.43 mmol) der Verbindung aus Beispiel 12b) und 538 µl (4.27 mmol) Chlorameisensäure-*n*-butylester 270 mg der gewünschten Titelverbindung in Form eines blaßgelben, amorphen Schaums.
R_{f} (SiO₂, EE/n-Heptan 1: 1) = 0.14
MS (ESI): m/z = 633 [M+H]⁺

### Beispiel 13

### 2-N-(2-Furanoyl)-2-N-[4-[2-(n-butyloxycarbonylsulfonamido)-5-isobutyl-3-thienyl]-benzyl]-amino-3-methyl-L-buttersäure

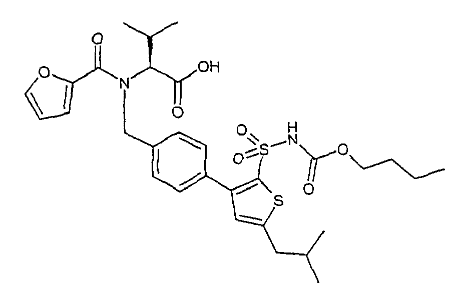

Die Darstellung der Titelverbindung erfolgte durch Behandlung der Verbindung aus Beispiel 12c) mit Natronlauge nach dem in Beispiel 6) angeführten Verfahren. Dabei resultierten aus 140 mg (0.22 mmol) der Verbindung aus Beispiel 12c) 122 mg der gewünschten Titelverbindung in Form eines weißen Feststoffs.
Fp: 93 °C
R_{f} (SiO₂, CH₂Cl₂/Methanol 18:2) = 0.23
MS (ESI): m/z = 619 [M+H]⁺

### Beispiel 14

### 2-N-(2-Furanoyl)-2-N-[4-[2-(methylaminocarbonylsulfonamido)-5-isobutyl-3-thienyl]-benzyl]-amino-3-methyl-L-buttersäuremethylester

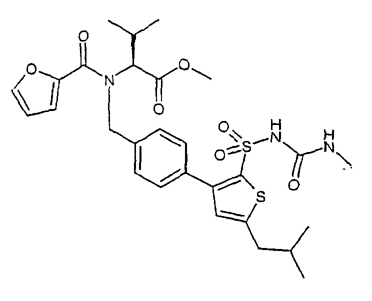

Eine Lösung aus 230 mg (0.43 mmol) der Verbindung aus Beispiel 12b) in 4 mL DMSO wurde mit 83.6 mg (0.47 mmol) N-Methyl-2,2,2-trichloracetamid und 52.6 mg (1.30 mmol) gepulvertem NaOH versetzt und 1.5 h bei 80 °C gerührt. Die Reaktionslösung wurde abgekühlt, mit Eis versetzt und der pH durch Zugabe von 2 N Salzsäure auf 4 eingestellt. Der dabei ausgefallene Niederschlag wurde abgesaugt, mit Wasser gewaschen, getrocknet und durch Chromatographie an SiO₂ mit EE/Heptan (4:1) als Laufmittel gereinigt. Es wurde 210 mg der Titelverbindung in Form eines weißen Feststoffes gewonnen.
Fp: 84 °C
R_{f} (SiO₂, EE/*n*-Heptan 4:1) = 0.12
MS (FAB): m/z = 590 [M+H]⁺

### Beispiel 15

### 2-N-(2-Furanoyl)-2-N-[4-[2-(methylaminocarbonylsulfonamido)-5-isobutyl-3-thienyl]-benzyl]-amino-3-methyl-L-buttersäure

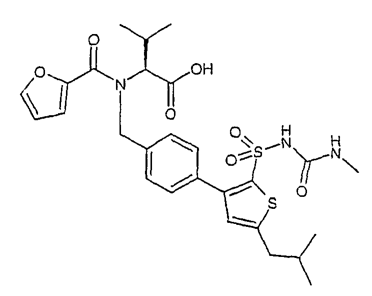

Die Darstellung der Titelverbindung erfolgte durch Behandlung der Verbindung aus Beispiel 14) mit Natronlauge nach dem in Beispiel 6) angeführten Verfahren. Dabei resultierten aus 150 mg (0.25 mmol) der Verbindung aus Beispiel 14) 136 mg der gewünschten Titelverbindung in Form eines weißen Feststoffs.
Fp: 134°C
R_{f} (SiO₂, EE/*n*-Heptan 1:1) = 0.14
MS (FAB): m/z = 576 [M+H]⁺

### Beispiel 16

### 2-N-(3-Methoxycarbonylpropionyl)-2-N-[4-[2-(n-butyloxycarbonylsulfonamido)-5-isobutyl-3-thienyl]-benzyl]-amino-3-methyl-L-buttersäuremethylester

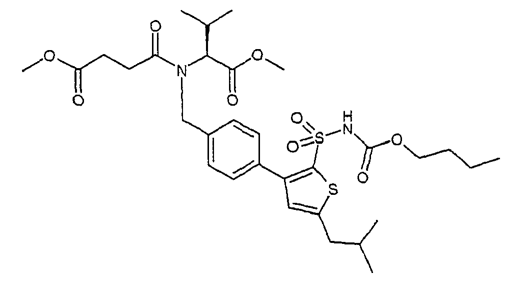

### a) 2-N-(3-Methoxycarbonylpropionyl)-2-N-[4-[2-(N-tert.-butyl)-sulfonamido-5-isobutyl-3-thienyl]-benzyl]-amino-3-methyl-L-buttersäuremethylester

Die Herstellung der Titelverbindung erfolgte durch Umsatz der Verbindung aus Beispiel 1e) mit 3-Carbomethoxypropionylchlorid nach dem in Beispiel 1f) angeführten Verfahren. Dabei resultierten aus 750 mg (1.52 mmol) der Verbindung aus Beispiel 1e) 904 mg der gewünschten Titelverbindung als blaßgelbes Öl.
R_{f} (SiO₂, EE/*n*-Heptan 1:3) = 0.18
MS (ESI): m/z = 609 [M+H]⁺

### b) 2-N-(3-Methoxycarbonylpropionyl)-2-N-[4-[2-sulfonamido-5-isobutyl-3-thienyl]-benzyl]-amino-3-methyl-L-buttersäuremethylester

Die Herstellung der Titelverbindung erfolgte durch Behandlung der Verbindung aus Beispiel 16a) mit Trifluoressigsäure nach dem in Beispiel 1g) angeführten Verfahren. Dabei resultierten aus 890 mg (1.46 mmol) der Verbindung aus Beispiel 16a) 510 mg der gewünschten Titelverbindung in Form eines weißen Feststoffs.
Fp: 66 °C
R_{f} (SiO₂, EE/*n*-Heptan 1:1) = 0.20
MS (ESI): m/z = 553 [M+H]⁺

### c) 2-N-(3-Methoxycarbonylpropionyl)-2-N-[4-[2-(n-butyloxycarbonylsulfonamido)-5-isobutyl-3-thienyl]-benzyl]-amino-3-methyl-L-buttersäuremethylester

Die Herstellung der Titelverbindung erfolgte durch Umsatz der Verbindung aus Beispiel 16b) mit Chlorameisensäure-*n*-butylester nach dem in Beispiel 1h) angeführten Verfahren. Dabei resultierten aus 240 mg (0.43 mmol) der Verbindung aus Beispiel 16b) und 547 µl (4.34 mmol) Chlorameisensäure-*n*-butylester 260 mg der gewünschten Titelverbindung in Form eines blaßgelben, amorphen Schaums.
R_{f} (SiO₂, EE/*n*-Heptan 1:1) = 0.11
MS (ESI): m/z = 653 [M+H]⁺

### Beispiel 17

### 2-N-(3-Carboxypropionyl)-2-N-[4-[2-(n-butyloxycarbonylsulfolnamido)-5-isobutyl-3-thienyl]-benzyl]-amino-3-methyl-L-buttersäure

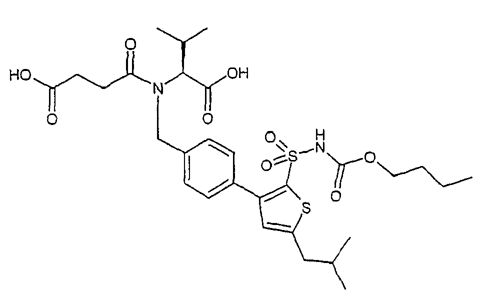

Die Darstellung der Titelverbindung erfolgte durch Behandlung der Verbindung aus Beispiel 16c) mit Natronlauge nach dem in Beispiel 6) angeführten Verfahren. Dabei resultierten aus 208 mg (0.32 mmol) der Verbindung aus Beispiel 16c) 170 mg der gewünschten Titelverbindung in Form eines weißen Feststoffs.
Fp: 66 °C
R_{f} (SiO₂, EE/*n*-Heptan 4:1) = 0.05
MS (ESI): m/z = 625 [M+H]⁺

### Beispiel 18

### 2-N-(3-Methoxycarbonylpropionyl)-2-N-[4-(2-(ethylaminocarbonylsulfonamido)-5-isobutyl-3-thienyl]-benzyl]-amino-3-methyl-L-buttersäuremethylester

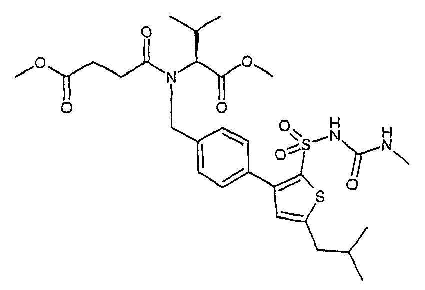

Die Titelverbindung wurde aus der Verbindung aus Beispiel 16b) nach dem in Beispiel 14) angeführten Verfahren hergestellt. Es resultierten aus 238 mg (0.43 mmol) der Verbindung aus Beispiel 16b) 180 mg der Titelverbindung als weißer Feststoff.
Fp: 158 °C
R_{f} (SiO₂, EE/*n*-Heptan 4:2) = 0.09
MS (FAB): m/z = 610 [M+H]⁺

### Beispiel 19

### 2-N-(3-Carboxypropionyl)-2-N-[4-[2-(ethylaminocarbonylsulfonamido)-5-isobutyl-3-thienyl]-benzyl]-amino-3-methyl-L-buttersäure

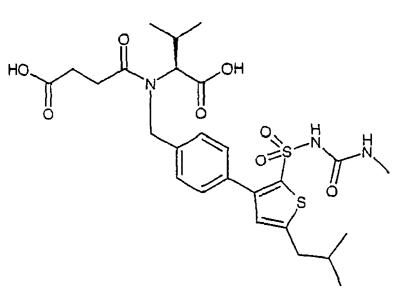

Die Darstellung der Titelverbindung erfolgte durch Behandlung der Verbindung aus Beispiel 18) mit Natronlauge nach dem in Beispiel 6) angeführten Verfahren. Dabei resultierten aus 125 mg (0.21 mmol) der Verbindung aus Beispiel 18) 105 mg der gewünschten Titelverbindung in Form eines weißen Feststoffs.
Fp: 128 °C
R_{f} (SiO₂, CH₂Cl₂/MeOH 18:2) = 0.23
MS (ESI): m/z = 582 [M+H]⁺

### Beispiel 20

### 2-N-Benzoyl-2-N-[4-[2-(ethylaminocarbonylsulfonamido)-5-isobutyl-3-thienyl]-benzyl]-amino-2S-phenyl-essigsäuremethylester

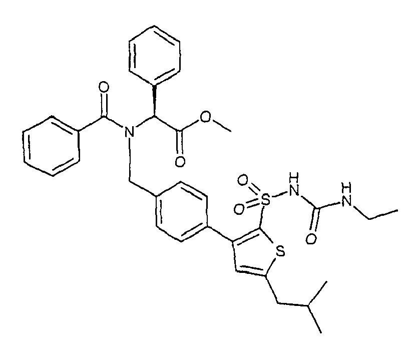

### a) 2-N-[4-[2-(N-tert.-Butyl-sulfonamido)-5-isobutyl-3-thienyl]-benzyl]-amino-2S-phenylessigsäuremethylester

Die Herstellung der Titelverbindung erfolgte durch Umsatz der Verbindung aus Beispiel 1d) mit S-(+)-2-Phenylglycinmethylester-Hydrochlorid nach dem in Beispiel 1e) angeführten Verfahren. Dabei resultierten aus 500 mg (1.32 mmol) der Verbindung aus Beispiel 1d) und 530.4 mg (2.63 mmol) S-(+)-2-Phenylglycinmethylester-Hydrochlorid 387 mg der gewünschten Titelverbindung in Form eines schwach gelbgefärbten Öls.
R_{f} (SiO₂, EE/*n*-Heptan 1:1) = 0.32
MS (ESI): m/z = 529 [M+H]⁺

### b) 2-N-Benzoyl-2-N-[4-[2-(N-tert.-Butyl-sulfonamido)-5-isobutyl-3-thienyl]-benzyl]-amino-2S-phenyl-essigsäuremethylester

Die Herstellung der Titelverbindung erfolgte durch Umsatz der Verbindung aus Beispiel 20a) mit Benzoylchlorid nach dem in Beispiel 1f) angeführten Verfahren. Dabei resultierten aus 193 mg (0.37 mmol) der Verbindung aus Beispiel 20a) und 63.8 µl (0.55 mmol) Benzoylchlorid 228 mg der gewünschten Titelverbindung in Form eines weißen, amorphen Schaums.
R_{f} (SiO₂, EE/*n*-Heptan 1:4) = 0.12
MS (ESI): m/z = 633 [M+H]⁺

### c) 2-N-Benzoyl-2-N-[4-[2-sulfonamido-5-isobutyl-3-thienyl]-benzyl]-amino-2S-phenylessigsäuremethylester

Die Herstellung der Titelverbindung erfolgte durch Behandlung der Verbindung aus Beispiel 20b) mit Trifluoressigsäure nach dem in Beispiel 1g) angeführten Verfahren. Dabei resultierten aus 220 mg (0.35 mmol) der Verbindung aus Beispiel 20b) 132 mg der gewünschten Titelverbindung in Form eines weißen, amorphen Schaums.
R_{f} (SiO₂, EE/*n*-Heptan 1:1) = 0.26
MS (ESI): m/z = 577 [M+H]⁺

### d) 2-N-Benzoyl-2-N-[4-[2-(ethylaminocarbonylsulfonamido)-5-isobutyl-3-thienyl]-benzyl]-amino-2S-phenyl-essigsäuremethylester

Die Herstellung der Titelverbindung erfolgte durch Umsatz der Verbindung aus Beispiel 20c) mit Ethylisocyanat nach dem in Beispiel 4) angeführten Verfahren. Dabei resultierten aus 128 mg (0.22 mmol) der Verbindung aus Beispiel 20c) 88 mg der Titelverbindung in Form eines weißen, amorphen Feststoffs.
R_{f} (SiO₂, EE/n-Heptan 4:1) = 0.35
MS (ESI): m/z = 648 [M+H]⁺

### Beispiel 21

### 2-N-Acetyl-2-N-[4-[2-(ethylaminocarbonylsulfonamido)-5-isobutyl-3-thienyl]-benzyl]-amino-2S-phenyl-essigsäuremethylester

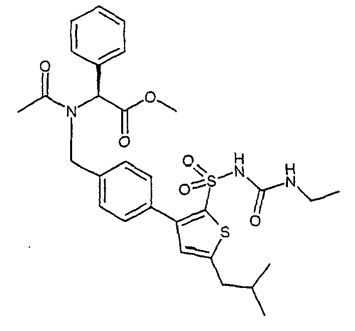

### a) 2-N-Acetyl-2-N-[4-[2-(N-tert.-Butyl-sulfonamido)-5-isobutyl-3-thienyl]-benzyl]-amino-2S-phenyl-essigsäuremethylester

Die Herstellung der Titelverbindung erfolgte durch Umsatz der Verbindung aus Beispiel 20a) mit Acetylchlorid nach dem in Beispiel 1f) angeführten Verfahren. Dabei resultierten aus 193 mg (0.37 mmol) der Verbindung aus Beispiel 20a) und 39.1 µl (0.55 mmol) Acetylchlorid 137 mg der gewünschten Titelverbindung in Form eines blaßgelben Öls.
R_{f} (SiO₂, EE/*n*-Heptan 1:4) = 0.17
MS (ESI): m/z = 571 [M+H]⁺

### b) 2-N-Acetyl-2-N-[4-[2-sulfonamido-5-isobutyl-3-thienyl]-benzyl]-amino-2S-phenylessigsäuremethylester

Die Herstellung der Titelverbindung erfolgte durch Behandlung der Verbindung aus Beispiel 21a) mit Trifluoressigsäure nach dem in Beispiel 1g) angeführten Verfahren. Dabei resultierten aus 134 mg (0.24 mmol) der Verbindung aus Beispiel 21a) 68 mg der gewünschten Titelverbindung in Form eines weißen, amorphen Schaums.
R_{f} (SiO₂, EE/*n*-Heptan 1:1) = 0.10
MS (ESI): m/z = 515 [M+H]⁺

### c) 2-N-Acetyl-2-N-[4-[2-(ethylaminocarbonylsulfonamido)-5-isobutyl-3-thienyl]-benzyl]-amino-2S-phenyl-essigsäuremethylester

Die Herstellung der Titelverbindung erfolgte durch Umsatz der Verbindung aus Beispiel 21b) mit Ethylisocyanat nach dem in Beispiel 4) angeführten Verfahren. Dabei resultierten aus 66 mg (0.13 mmol) der Verbindung aus Beispiel 21b) 75 mg der Titelverbindung in Form eines weißen, amorphen Feststoffs.
R_{f} (SiO₂, EE/n-Heptan 4: 1) = 0.10
MS (ESI): m/z = 586 [M+H]⁺

### Beispiel 22

### 2-N-Benzoyl-2-N-[4-[2-(ethylaminocarbonylsulfonamido)-5-isobutyl-3-thienyl]-benzyl]-amino-2S-cyclohexyl-essigsäuremethylester

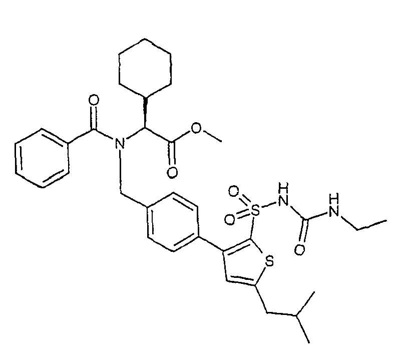

### a) 2-N-[4-[2-(N-tert.-Butyl-sulfonamido)-5-isobutyl-3-thienyl]-benzyl]-amino-2S-cyclohexyl-essigsäuremethylester

Die Herstellung der Titelverbindung erfolgte durch Umsatz der Verbindung aus Beispiel 1d) mit S-2-Amino-2-cyclohexyl-essigsäuremethylester-Hydrochlorid nach dem in Beispiel 1e) angeführten Verfahren. Dabei resultierten aus 500 mg (1.32 mmol) der Verbindung aus Beispiel 1d) und 546.2 mg (2.63 mmol) S-2-Amino-2-cyclohexylessigsäuremethylester-Hydrochlorid 404 mg der gewünschten Titel-verbindung in Form eines schwach gelbgefärbten Öls.
R_{f} (SiO₂, EE/*n*-Heptan 1:4) =0.17
MS (ESI): m/z = 535 [M+H]⁺

### b) 2-N-Benzoyl-2-N-[4-[2-(N-tert.-Butyl-sulfonamido)-5-isobutyl-3-thienyl]-benzyl]-amino-2S-cyclohexyl-essigsäuremethylester

Die Herstellung der Titelverbindung erfolgte durch Umsatz der Verbindung aus Beispiel 22a) mit Benzoylchlorid nach dem in Beispiel 1f) angeführten Verfahren. Dabei resultierten aus 200 mg (0.37 mmol) der Verbindung aus Beispiel 22a) und 65.9 µl (0.57 mmol) Benzoylchlorid 241 mg der gewünschten Titelverbindung in Form eines weißen, amorphen Schaums.
R_{f} (SiO₂, EE/*n*-Heptan 1:4) = 0.21
MS (ESI): m/z = 639 [M+H]⁺

### c) 2-N-Benzoyl-2-N-[4-[2-sulfonamido-5-isobutyl-3-thienyl]-benzyl]-amino-2S-cyclohexyl-essigsäuremethylester

Die Herstellung der Titelverbindung erfolgte durch Behandlung der Verbindung aus Beispiel 22b) mit Trifluoressigsäure nach dem in Beispiel 1g) angeführten Verfahren. Dabei resultierten aus 235 mg (0.37 mmol) der Verbindung aus Beispiel 22b) 130 mg der gewünschten Titelverbindung in Form eines weißen, amorphen Schaums.
R_{f} (SiO₂, EE/*n*-Heptan 1:1) = 0.37
MS (ESI): m/z = 583 [M+H]⁺

### d) 2-N-Benzoyl-2-N-[4-[2-(ethylaminocarbonylsulfonamido)-5-isobutyl-3-thienyl]-benzyl]-amino-2S-cyclohexyl-essigsäuremethylester

Die Herstellung der Titelverbindung erfolgte durch Umsatz der Verbindung aus Beispiel 22c) mit Ethylisocyanat nach dem in Beispiel 4) angeführten Verfahren. Dabei resultierten aus 125 mg (0.22 mmol) der Verbindung aus Beispiel 22c) 90 mg der Titelverbindung in Form eines weißen, amorphen Feststoffs.
R_{f} (SiO₂, EE/n-Heptan 4:1) = 0.37
MS (ESI): m/z = 654 [M+H]⁺

### Beispiel 23

### 2-N-Acetyl-2-N-[4-[2-(ethylaminocarbonylsulfonamido)-5-isobutyl-3-thienyl]-benzyl]-amino-2S-cyclohexyl-essigsäuremethylester

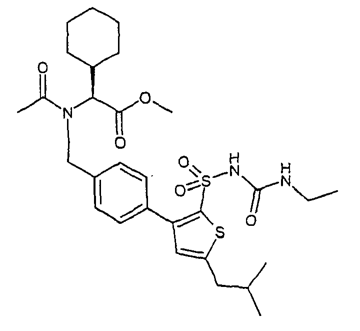

### a) 2-N-Acetyl-2-N-[4-[2-(N-tert.-Butyl-sulfonamido)-5-isobutyl-3-thienyl]-benzyl]-amino-2S-cyclohexyl-essigsäuremethylester

Die Herstellung der Titelverbindung erfolgte durch Umsatz der Verbindung aus Beispiel 22a) mit Acetylchlorid nach dem in Beispiel 1f) angeführten Verfahren. Dabei resultierten aus 200 mg (0.37 mmol) der Verbindung aus Beispiel 22a) und 40.4 µl (0.57 mmol) Acetylchlorid 117 mg der gewünschten Titelverbindung in Form eines gelben Öls.
R_{f} (SiO₂, EE/*n*-Heptan 1:4) = 0.25
MS (ESI): m/z = 577 [M+H]⁺

### b) 2-N-Acetyl-2-N-[4-[2-sulfonamido-5-isobutyl-3-thienyl]-benzyl]-amino-2S-cydohexylessigsäuremethylester

Die Herstellung der Titelverbindung erfolgte durch Behandlung der Verbindung aus Beispiel 23a) mit Trifluoressigsäure nach dem in Beispiel 1g) angeführten Verfahren. Dabei resultierten aus 114 mg (0.20 mmol) der Verbindung aus Beispiel 23a) 75 mg der gewünschten Titelverbindung in Form eines blaßgelben, amorphen Schaums.
R_{f} (SiO₂, EE/*n*-Heptan 1:1) = 0.16
MS (ESI): m/z = 521 [M+H]⁺

### c) 2-N-Acetyl-2-N-[4-[2-(ethylaminocarbonylsulfonamido)-5-isobutyl-3-thienyl]-benzyl]-amino-2S-cyclohexyl-essigsäuremethylester

Die Herstellung der Titelverbindung erfolgte durch Umsatz der Verbindung aus Beispiel 23b) mit Ethylisocyanat nach dem in Beispiel 4) angeführten Verfahren. Dabei resultierten aus 72 mg (0.14 mmol) der Verbindung aus Beispiel 23b) 77 mg der Titelverbindung in Form eines weißen, amorphen Feststoffs.
R_{f} (SiO₂, EE/n-Heptan 2:1) = 0.18
MS (ESI): m/z = 592 [M+H]⁺

### Beispiel 24

### 2-Cyclohexyl-N-(2-methoxyethyl)-N-[4-[2-(n-butyloxycarbonylsulfonamido)-5-isobutyl-3-thienyl]-benzyl]-acetamid

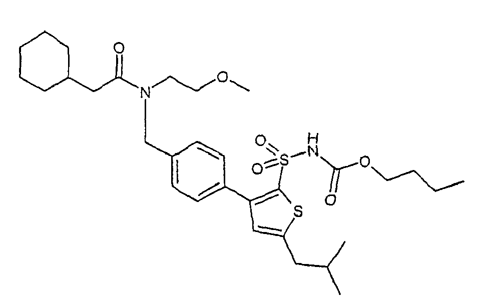

### a) N-[4-[2-(N-tert.-Butyl-sulfonamido)-5-isobutyl-3-thienyl]-benzyl]-2-methoxy-ethylamin

Die Herstellung der Titelverbindung erfolgte durch Umsatz der Verbindung aus Beispiel 1d) mit 2-Methoxyethylamin nach dem in Beispiel 1e) angeführten Verfahren. Dabei resultierten aus 600 mg (1.58 mmol) der Verbindung aus Beispiel 1d) und 275 µl (1.58 mmol) 2-Methoxyethylamin 342 mg der gewünschten Titel-verbindung in Form eines schwach gelbgefärbten, amorphen Schaums.
R_{f} (SiO₂, EE/*n*-Heptan 1:1) = 0.05
MS (ESI): m/z = 439 [M+H]⁺

### b) 2-Cyclohexyl-N-(2-methoxyethyl)-N-[4-[2-(N-tert.-butyl-sulfonamido)-5-isobutyl-3-thienyl]-benzyl]-acetamid

Eine Lösung aus 155 mg (0.36 mmol) der Verbindung aus Beispiel 24a), 51 µl (0.36 mmol) 2-Cyclohexylessigsäure, 125 µl (0.72 mmol) N-Ethyl-diisopropylamin und 120 mg (0.36 mmol) O-(Cyan)-(ethoxycarbonyl)-methylenamino-1,1,3,3-tetramethyluronium-tetrafluoroborat (TOTU) in 8 ml abs. DMF wurde für 2 h an RT gerührt. Anschließend wurde das Lösungsmittel im Vakuum entfernt, der verbliebene Rückstand in CH₂Cl₂/Wasser (1:1) aufgenommen und die organische Phase abgetrennt. Nach Trocknung über Na₂SO₄, Einengung und chromatographische Reinigung des verbliebenen Rückstandes an SiO₂ mit EE/*n*-Heptan als Laufmittel resultierten 185 mg der gewünschten Titelverbindung in Form eines hellgelben Öls.
R_{f} (SiO₂, EE/*n*-Heptan 1:1) = 0.27
MS (ESI): m/z = 563 [M+H]⁺

### c) 2-Cyclohexyl-N-(2-methoxyethyl)-N-[4-[2-sulfonamido-5-isobutyl-3-thienyl]-benzyl]-acetamid

Die Herstellung der Titelverbindung erfolgte durch Behandlung der Verbindung aus Beispiel 24b) mit Trifluoressigsäure nach dem in Beispiel 1g) angeführten Verfahren. Dabei resultierten aus 183 mg (0.32 mmol) der Verbindung aus Beispiel 24b) 108 mg der gewünschten Titelverbindung in Form eines weißen, amorphen Schaums.
R_{f} (SiO₂, EE/*n*-Heptan 1:1) = 0.13
MS (ESI): m/z = 507 [M+H]⁺

### d) 2-Cyclohexyl-N-(2-methoxyethyl)-N-[4-[2-(n-butyloxycarbonylsulfonamido)-5-isobutyl-3-thienyl]-benzyl]-acetamid

Die Herstellung der Titelverbindung erfolgte durch Umsatz der Verbindung aus Beispiel 24c) mit Chlorameisensäure-*n*-butylester nach dem in Beispiel 1h) angeführten Verfahren. Dabei resultierten aus 50 mg (0.10 mmol) der Verbindung aus Beispiel 24c) und 124 µl (1.00 mmol) Chlorameisensäure-*n*-butylester 46 mg der gewünschten Titelverbindung in Form eines blaßgelben, amorphen Schaums.
R_{f} (SiO₂, EE/*n*-Heptan 1:1) = 0.14
MS (ESI): m/z = 607 [M+H]⁺

### Beispiel 25

### 2-Cyclohexyl-N-(2-methoxyethyl)-N-[4-[2-(ethylaminocarbonylsulfonamido)-5-isobutyl-3-thienyl]-benzyl]-acetamid

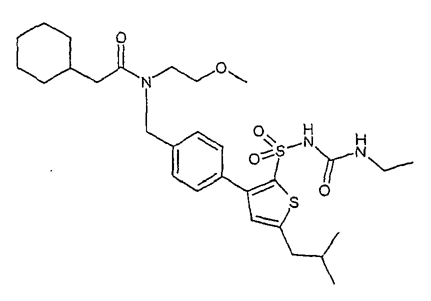

Die Herstellung der Titelverbindung erfolgte durch Umsatz der Verbindung aus Beispiel 24c) mit Ethylisocyanat nach dem in Beispiel 4) angeführten Verfahren. Dabei resultierten aus 58 mg (0.11 mmol) der Verbindung aus Beispiel 24c) 60 mg der Titelverbindung in Form eines weißen Feststoffs.
Fp: 95° C
R_{f} (SiO₂, EE/n-Heptan 1:1) = 0.06
MS (ESI): m/z = 578 [M+H]⁺

### Beispiel 26

### 2-Cyclohexyl-N-cyclopropyl-N-[4-[2-(n-butyloxycarbonylsulfonamido)-5-isobutyl-3-thienyl]-benzyl]-acetamid

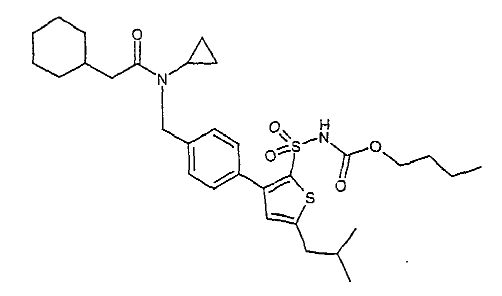

### a) N-[4-[2-(N-tert.-Butyl-sulfonamido)-5-isobutyl-3-thienyl]-benzyl]-cyclopropylamin

Die Herstellung der Titelverbindung erfolgte durch Umsatz der Verbindung aus Beispiel 1d) mit Cyclopropylamin nach dem in Beispiel 1e) angeführten Verfahren. Dabei resultierten aus 1.20 g (3.16 mmol) der Verbindung aus Beispiel 1d) und 442 µl (6.32 mmol) Cyclopropylamin 852 mg der gewünschten Titelverbindung in Form eines schwach gelbgefärbten, amorphen Schaums.
R_{f} (SiO₂, EE/*n*-Heptan 1:1) = 0.10
MS (ESI): m/z = 421 [M+H]⁺

### b) 2-Cyclohexyl-N-cyclopropyl-N-[4-[2-(N-tert.-butyl-sulfonamido)-5-isobutyl-3-thienyl]-benzyl]-acetamid

Die Herstellung der Titelverbindung erfolgte durch Umsatz der Verbindung aus Beispiel 26a) mit 2-Cyclohexylessigsäure nach dem in Beispiel 24b) angeführten Verfahren. Dabei resultierten aus 150 mg (0.36 mmol) der Verbindung aus Beispiel 26a) 172 mg der gewünschten Titelverbindung als weißer, amorpher Schaum.
R_{f} (SiO₂, EE/*n*-Heptan 1:1) = 0.38
MS (ESI): m/z = 545 [M+H]⁺

### c) 2-Cyclohexyl-N-cyclopropyl-N-[4-[2-sulfonamido-5-isobutyl-3-thienyl]-benzyl]-acetamid

Die Herstellung der Titelverbindung erfolgte durch Behandlung der Verbindung aus Beispiel 26b) mit Trifluoressigsäure nach dem in Beispiel 1g) angeführten Verfahren. Dabei resultierten aus 170 mg (0.31 mmol) der Verbindung aus Beispiel 26b) 120 mg der gewünschten Titelverbindung in Form eines weißen, amorphen Schaums.
R_{f} (SiO₂, EE/*n*-Heptan 1:1) = 0.24
MS (ESI): m/z = 489 [M+H]⁺

### d) 2-Cyclohexyl-N-cyclopropyl-N-[4-[2-(n-butyloxycarbonylsulfonamido)-5-isobutyl-3-thienyl]-benzyl]-acetamid

Die Herstellung der Titelverbindung erfolgte durch Umsatz der Verbindung aus Beispiel 26c) mit Chlorameisensäure-*n*-butylester nach dem in Beispiel 1h) angeführten Verfahren. Dabei resultierten aus 60 mg (0.12 mmol) der Verbindung aus Beispiel 26c) und 154 µl (1.23 mmol) Chlorameisensäure-*n*-butylester 66 mg der gewünschten Titelverbindung in Form eines blaßgelben, amorphen Schaums.
R_{f} (SiO₂, EE/n-Heptan 1:1) = 0.24
MS (ESI): m/z = 589 [M+H]⁺

### Beispiel 27

### 2-Cyclohexyl-N-cyclopropyl-N-[4-[2-(ethylaminocarbonylsulfonamido)-5-isobutyl-3-thienyl]-benzyl]-acetamid

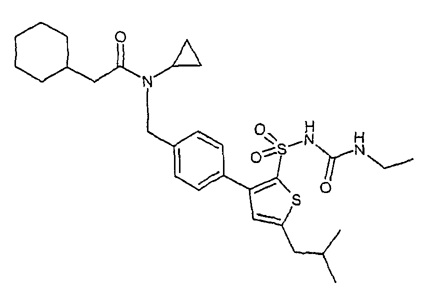

Die Herstellung der Titelverbindung erfolgte durch Umsatz der Verbindung aus Beispiel 26c) mit Ethylisocyanat nach dem in Beispiel 4) angeführten Verfahren. Dabei resultierten aus 60 mg (0.12 mmol) der Verbindung aus Beispiel 26c) 66 mg der Titelverbindung in Form eines weißen Feststoffs.
Fp: 103° C
R_{f} (SiO₂, EE/n-Heptan 1:1) = 0.17
MS (ESI): m/z = 560 [M+H]⁺

## Patentansprüche

1. Verbindungen der Formel (I) 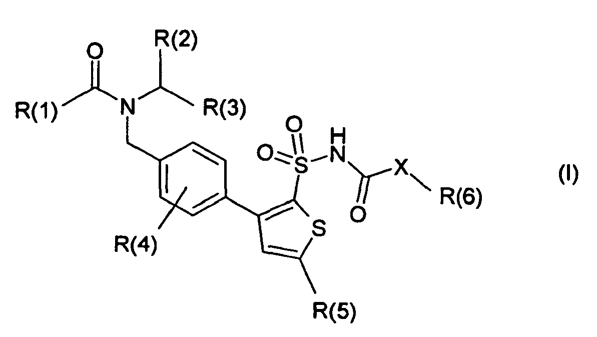
in denen die angeführten Reste die folgende Bedeutung haben:
R(1)
1. (C₁-C₅)-Alkyl, unsubstituiert oder substituiert mit einem Rest aus der Reihe NH₂, Halogen, O-(C₁-C₃)-Alkyl, CO-O-(C₁-C₃)-Alkyl und CO₂H;
2. (C₃-C₈)-Cycloalkyl;
3. (C₁-C₃)-Alkyl-(C₃-C₈)-Cycloalkyl;
4. (C₆-C₁₀)-Aryl, unsubstituiert oder substituiert mit einem Rest aus der Reihe Halogen und O-(C₁-C₃)-Alkyl;
5. (C₁-C₃)-Alkyl-(C₆-C₁₀)-Aryl, wobei der Arylrest unsubstituiert oder substituiert ist mit einem Rest aus der Reihe Halogen und O-(C₁-C₃)-Alkyl;
6. (C₁-C₅)-Heteroaryl; und
7. (C₁-C₃)-Alkyl-(C₁-C₅)-Heteroaryl;
R(2)
1. Wasserstoff;
2. (C₁-C₆)-Alkyl, unsubstituiert oder substituiert mit einem Rest aus der Reihe Halogen und O-(C₁-C₃)-Alkyl;
3. (C₃-C₈)-Cycloalkyl;
4. (C₁-C₃)-Alkyl-(C₃-C₈)-Cycloalkyl;
5. (C₆-C₁₀)-Aryl, unsubstituiert oder substituiert mit einem Rest aus der Reihe Halogen, O-(C₁-C₃)-Alkyl und CO-O-(C₁-C₃)-Alkyl; und
6. (C₁-C₃)-Alkyl-(C₆-C₁₀)-Aryl, unsubstituiert oder substituiert mit einem Rest aus der Reihe Halogen und O-(C₁-C₃)-Alkyl;
R(3)
1. Wasserstoff;
2. COOH; und
3. COO-(C₁-C₄)-Alkyl;
R(4)
1. Wasserstoff;
2. Halogen; und
3. (C₁-C₄)-Alkyl;
R(5)
1. Wasserstoff und
2. (C₁-C₆)-Alkyl;
R(6)
1. Wasserstoff;
2. (C₁-C₆)-Alkyl;
3. (C₁-C₃)-Alkyl-(C₃-C₈)-Cycloalkyl; und
4. (C₂-C₆)-Alkenyl;
X
1. Sauerstoff und
2. NH;
in allen ihren stereoisomeren Formen und Mischungen davon in allen Verhältnissen, ihre Solvate und ihre physiologisch verträglichen Salze.

2. Verbindungen der Formel (I) gemäß Anspruch 1, in denen bedeutet:
R(1)
1. (C₁-C₅)-Alkyl, unsubstituiert oder substituiert mit einem Rest aus der Reihe NH₂, Halogen, O-(C₁-C₃)-Alkyl, CO-O-(C₁-C₃)-Alkyl und CO₂H;
2. (C₃-C₆)-Cycloalkyl;
3. (C₁-C₃)-Alkyl-(C₃-C₆)-Cycloalkyl;
4. (C₆-C₁₀)-Aryl, unsubstituiert oder substituiert mit einem Rest aus der Reihe Halogen und O-(C₁-C₃)-Alkyl;
5. (C₁-C₃)-Alkyl-(C₆-C₁₀)-Aryl, wobei der Arylrest unsubstituiert oder substituiert ist mit einem Rest aus der Reihe Halogen und O-(C₁-C₃)-Alkyl;
6. (C₃-C₅)-Heteroaryl; und
7. (C₁-C₃)-Alkyl-(C₃-C₅)-Heteroaryl;
R(2)
1. Wasserstoff;
2. (C₁-C₆)-Alkyl, unsubstituiert oder substituiert mit einem Rest aus der Reihe Halogen und O-(C₁-C₃)-Alkyl;
3. (C₃-C₆)-Cycloalkyl;
4. (C₁-C₃)-Alkyl-(C₃-C₆)-Cycloalkyl;
5. (C₆-C₁₀)-Aryl, unsubstituiert oder substituiert mit einem Rest aus der Reihe Halogen, O-(C₁-C₃)-Alkyl und CO-O-(C₁-C₃)-Alkyl; und
6. (C₁-C₃)-Alkyl-(C₆-C₁₀)-Aryl, unsubstituiert oder substituiert mit einem Rest aus der Reihe Halogen und O-(C₁-C₃)-Alkyl;
R(3)
1. Wasserstoff;
2. COOH; und
3. COO-(C₁-C₄)-Alkyl;
R(4)
1. Wasserstoff;
2. Halogen; und
3. (C₁-C₄)-Alkyl;
R(5)
1. Wasserstoff und
2. (C₁-C₄)-Alkyl;
R(6)
1. Wasserstoff;
2. (C₁-C₄)-Alkyl;
3. (C₁-C₃)-Alkyl-(C₃-C₆)-Cycloalkyl; und
4. (C₃-C₅)-Alkenyl;
X
1. Sauerstoff und
2. NH;
in allen ihren stereoisomeren Formen und Mischungen davon in allen Verhältnissen, ihre Solvate und ihre physiologisch verträglichen Salze.

3. Verbindungen der Formel (I) gemäß Anspruch 1 oder 2, in denen bedeutet:
R(1)
1. (C₁-C₃)-Alkyl, unsubstituiert oder substituiert mit einem Rest aus der Reihe Fluor, Methoxy, Ethoxy, CO-O-(C₁-C₃)-Alkyl und CO₂H;
2. (C₁-C₃)-Alkyl-Cyclohexyl;
3. Phenyl, substituiert oder unsubstituiert mit einem Rest aus der Reihe Fluor und Methoxy;
4. (C₁-C₃)-Alkyl-Phenyl, wobei der Phenylrest unsubstituiert oder substituiert ist mit einem Rest aus der Reihe Fluor und Methoxy;
5. Furanyl, Thienyl oder Pyridyl;
R(2)
1. Wasserstoff;
2. (C₁-C₆)-Alkyl, unsubstituiert oder substituiert mit einem Rest aus der Reihe Fluor, Methoxy und Ethoxy;
3. Phenyl, unsubstituiert oder substituiert mit einem Rest aus der Reihe Fluor und Methoxy;
4. (C₁-C₆)-Cycloalkyl;
R(4)
1. Wasserstoff;
2. Methyl; und
3. Chlor;
R(5) (C₁-C₄)-Alkyl;
R(6) (C₁-C₄)-Alkyl;
und die Reste R(3) und X wie in den vorangegangen Ansprüchen definiert sind, in allen ihren stereoisomeren Formen und Mischungen davon ihre Solvate, und ihre physiologisch verträglichen Salze.

4. Verbindungen der Formel (I) gemäß einem oder mehreren der Ansprüche 1 bis 3, in denen bedeutet:
R(1)
1. (C₁-C₃)-Alkyl, unsubstituiert oder substituiert mit einem Rest aus der Reihe Fluor, Methoxy, Ethoxy, CO-O-(C₁-C₃)-Alkyl und CO₂H;
2. (C₁-C₃)-Alkyl-Cyclohexyl;
3. Phenyl, substituiert oder unsubstituiert mit einem Rest aus der Reihe Fluor und Methoxy;
4. (C₁-C₃)-Alkyl-Phenyl, wobei der Phenylrest unsubstituiert oder substituiert ist mit einem Rest aus der Reihe Fluor und Methoxy;
5. Furanyl, Thienyl oder Pyridyl;
R(2)
1. Wasserstoff;
2. (C₁-C₆)-Alkyl, unsubstituiert oder substituiert mit einem Rest aus der Reihe Fluor, Methoxy und Ethoxy;
3. Phenyl, unsubstituiert oder substituiert mit einem Rest aus der Reihe Fluor und Methoxy;
4. Cyclopropyl oder Cyclohexyl;
R(4)
1. Wasserstoff;
2. Methyl; und
3. Chlor;
R(5) Propyl und Butyl, insbesondere n-Propyl, iso-Propyl und 2-iso-Butyl;
R(6) Methyl, Ethyl, n-Propyl, iso-Propyl, n-Butyl und iso-Butyl;
und die Reste R(3) und X wie in den vorangegangenen Ansprüchen definiert sind, in allen ihren stereoisomeren Formen und Mischungen davon ihre Solvate, und ihre physiologisch verträglichen Salze.

5. Verbindungen der Formel (I) gemäß einem oder mehreren der Ansprüche 1 bis 4, worin diese Verbindungen der Formel (II) darstellen, 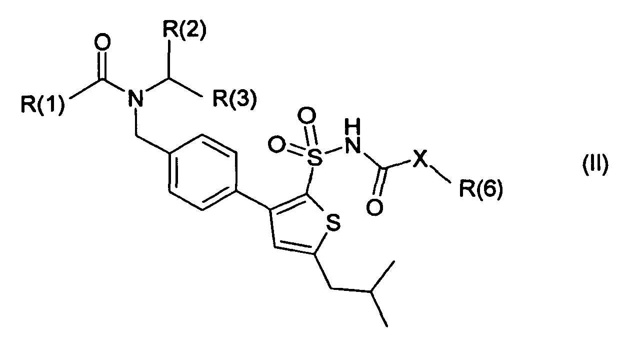
in der die Reste R(1), R(2), R(3), R(6) und X die in einem oder mehreren der Ansprüche 1 bis 4 angeführte Bedeutung haben, in allen ihren stereoisomeren Formen und Mischungen davon ihre Solvate, und ihre physiologisch verträglichen Salze.

6. Verbindungen der Formel (I) gemäß einem oder mehreren der Ansprüche 1 bis 5, **dadurch gekennzeichnet, dass** diese
2-*N* Benzoyl-2-*N*-[4-[2-(*n*-butyloxycarbonylsulfonamido)-5-isobutyl-3-thienyl]-benzyl]-amino-3-methyl-L-buttersäuremethylester;
2-*N*-Benzoyl-2-*N*-[4-[2-(*n*-butyloxycarbonylsulfonamido)-5-isobutyl-3-thienyl]-benzyl]-amino-3-methyl-L-buttersäureethylester;
2-*N*-Benzoyl-2-*N*-[4-[2-(*n*-butyloxycarbonylsulfonamido)-5-isobutyl-3-thienyl]-benzyl]-amino-3-methyl-L-buttersäure;
2-*N*-Benzoyl-2-*N*-[4-[2-(ethylaminocarbonylsulfonamido)-5-isobutyl-3-thienyl]-benzyl]-amino-3-methyl-L-buttersäuremethylester;
2-*N*-Benzoyl-2-*N*-[4-[2-(ethylaminocarbonylsulfonamido)-5-isobutyl-3-thienyl]-benzyl]-amino-3-methyl-L-buttersäureethylester;
2-*N*-Benzoyl-2-*N*-[4-[2-(ethylaminocarbonylsulfonamido)-5-isobutyl-3-thienyl]-benzyl]-amino-3-methyl-L-buttersäure;
2-*N*-Acetyl-2-*N*-[4-[2-(*n*-butyloxycarbonylsulfonamido)-5-isobutyl-3-thienyl]-benzyl]-amino-3-methyl-L-buttersäuremethylester;
2-*N*-Acetyl-2-*N*-[4-[2-(*n*-butyloxycarbonylsulfonamido)-5-isobutyl-3-thienyl]-benzyl]-amino-3-methyl-L-buttersäure;
2-*N*-Acetyl-2-*N*-[4-[2-(ethylaminocarbonylsulfonamido)-5-isobutyl-3-thienyl]-benzyl]-amino-3-methyl-L-buttersäuremethylester;
2-*N*-Acetyl-2-*N*-[4-[2-(ethylaminocarbonylsulfonamido)-5-isobutyl-3-thienyl]-benzyl]-amino-3-methyl-L-buttersäureethylester;
2-*N*-Acetyl-2-*N*-[4-[2-(ethylaminocarbonylsulfonamido)-5-isobutyl-3-thienyl]-benzyl]-amino-3-methyl-L-buttersäure;
2-*N*-(2-Furanoyl)-2-*N*-[4-[2-(*n*-butyloxycarbonylsulfonamido)-5-isobutyl-3-thienyl]-benzyl]-amino-3-methyl-L-buttersäuremethylester;
2-*N*-(2-Furanoyl)-2-*N*-[4-[2-(*n*-butyloxycarbonylsulfonamido)-5-isobutyl-3-thienyl]-benzyl]-amino-3-methyl-L-buttersäure;
2-*N*-(2-Furanoyl)-2-*N*-[4-[2-(methylaminocarbonylsulfonamido)-5-isobutyl-3-thienyl]-benzyl]-amino-3-methyl-L-buttersäuremethylester;
2-*N*-(2-Furanoyl)-2-*N*-[4-[2-(methylaminocarbonylsulfonamido)-5-isobutyl-3-thienyl]-benzyl]-amino-3-methyl-L-buttersäure;
2-*N*-(3-Methoxycarbonylpropionyl)-2-*N*-[4-[2-(*n*-butyloxycarbonylsulfonamido)-5-isobutyl-3-thienyl]-benzyl]-amino-3-methyl-L-buttersäuremethylester;
2-*N*-(3-Carboxypropionyl)-2-*N*-[4-[2-(*n*-butyloxycarbonylsulfonamido)-5-isobutyl-3-thienyl]-benzyl]-amino-3-methyl-L-buttersäure;
2-*N*-(3-Methoxycarbonylpropionyl)-2-*N*-[4-[2-(ethylaminocarbonylsulfonamido)-5-isobutyl-3-thienyl]-benzyl]-amino-3-methyl-L-buttersäuremethylester;
2-*N*-(3-Carboxypropionyl)-2-*N*-[4-[2-(ethylaminocarbonylsulfonamido)-5-isobutyl-3-thienyl]-benzyl]-amino-3-methyl-L-buttersäure;
2-*N*-Benzoyl-2-*N*-[4-[2-(ethylaminocarbonylsulfonamido)-5-isobutyl-3-thienyl]-benzyl]-amino-2S-phenyl-essigsäuremethylester;
2-*N*-Acetyl-2-*N*-[4-[2-(ethylaminocarbonylsulfonamido)-5-isobutyl-3-thienyl]-benzyl]-amino-2S-phenyl-essigsäuremethylester;
2-*N*-Benzoyl-2-*N*-[4-[2-(ethylaminocarbonylsulfonamido)-5-isobutyl-3-thienyl]-benzyl]-amino-2S-cyclohexyl-essigsäuremethylester;
2-*N*-Acetyl-2-*N*-[4-[2-(ethylaminocarbonylsulfonamido)-5-isobutyl-3-thienyl]-benzyl]-amino-2S-cyclohexyl-essigsäuremethylester;
2-Cyclohexyl-*N*-(2-methoxyethyl)-*N*-[4-[2-(*n*-butyloxycarbonylsulfonamido)-5-isobutyl-3-thienyl]-benzyl]-acetamid;
2-Cyclohexyl-*N*-(2-methoxyethyl)-*N*-[4-[2-(ethylaminocarbonylsulfonamido)-5-isobutyl-3-thienyl]-benzyl]-acetamid;
2-Cyclohexyl-*N*-cyclopropyl-*N*-[4-[2-(*n*-butyloxycarbonylsulfonamido)-5-isobutyl-3-thienyl]-benzyl]-acetamid; oder 2-Cuclohexyl-N-cyclopropyl-N-[4-[2-(ethylaminocarbonylsulfonamido)-5-isobutyl-3-thienyl]-benzyl]-acetamid;
sind sowie deren Solvate und deren physiologisch verträglichen Salze.

7. Verfahren zur Herstellung von Verbindungen der Formel (I) gemäß einem oder mehreren der Ansprüche 1 bis 6, **dadurch gekennzeichnet, daß** man
a) Thiophen-3-boronsäuren der Formel (III), 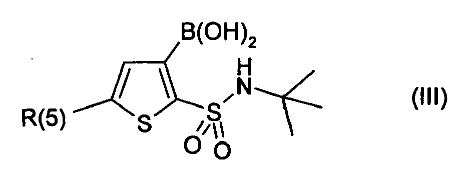
mit p-Brom-benzaldehyden der Formel (IV), 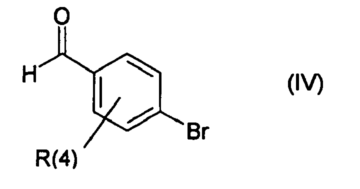
zu Verbindungen der Formel (V), 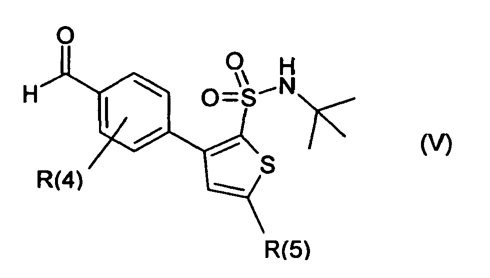
umsetzt;
b) die Verbindungen der Formel (V) mit primären Amino-Verbindungen der Formel (VI), 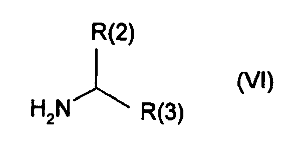
zu Verbindungen der Formel (VII), 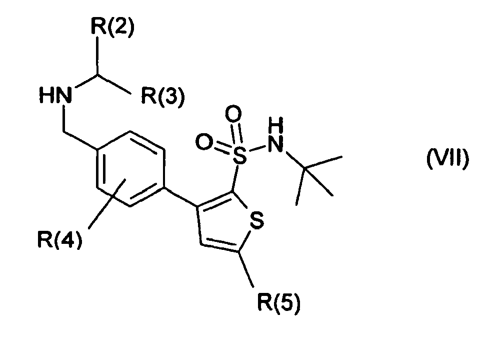
umsetzt;
c) die Verbindungen der Formel (VII) mit Acylchloriden des Typs R(1)-COCl zu Amiden der Formel (VIII) 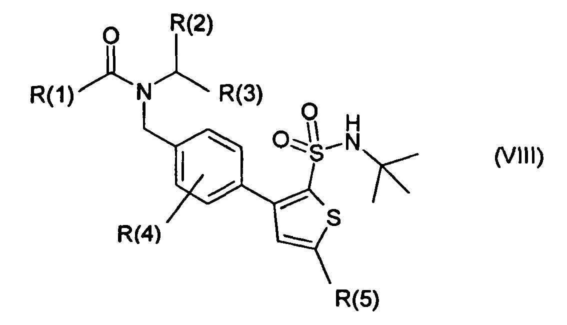
umsetzt;
d) die Verbindungen der Formel (VIII) durch Abspaltung der tert.-Butyl-Schutzgruppe in Sulfonamide der Formel (IX) überführt, und 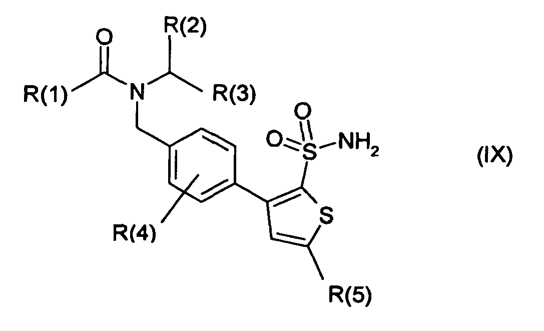
e) die Verbindungen der Formel (IX) durch Umsatz mit R(6)-substituierten Chlorameisensäureestern in die entsprechenden Sulfonylurethane der Formel (I) (X gleich O), oder durch Umsatz mit R(6)-substituierten Isocyanaten in die entsprechenden Sulfonylharnstoffe der Formel (I) (X gleich NH) überführt, wobei R(1), R(2), R(3) R(4) R(5) und R(6) wie in den Ansprüchen 1 bis 6 definiert sind.

8. Verbindungen der Formel (I) gemäß einem oder mehreren der Ansprüche 1 bis 7 und/oder ihre Solvate oder physiologisch verträglichen Salze zur Verwendung als Arzneimittel.

9. Pharmazeutische Zubereitung, **gekennzeichnet durch** einen wirksamen Gehalt einer Verbindung der Formel (I) nach einem oder mehreren der Ansprüche 1 bis 6 und/oder eines Solvats oder physiologisch verträglichen Salzes davon.

10. Pharmazeutische Zubereitung gemäß Anspruch 9, **gekennzeichnet durch** einen wirksamen Gehalt eines weiteren Wirkstoffes.

11. Verbindungen der Formel (I) gemäß einem oder mehreren der Ansprüche 1 bis 6 und/oder ihre Solvate oder physiologisch verträglichen Salze zur Stimulierung von Angiotensin(1-7) Rezeptoren.

12. Verbindungen der Formel (I) gemäß einem oder mehreren der Ansprüche 1 bis 6 und/oder ihre Solvate oder physiologisch verträglichen Salze zur Therapie und/oder Prävention von Krankheiten, die primär oder sekundär durch eine reduzierte Produktion und/oder Freisetzung der vasorelaxierenden, antithrombotischen und kardioprotektiven Botenstoffe cyclisches 3',5'-Guanosinmonophoshat (cGMP) und Stickstoffmonoxid (NO) verursacht oder zumindest mitverursacht werden.

13. Verbindungen der Formel (I) gemäß einem oder mehreren der Ansprüche 1 bis 6 und/oder ihre Solvate oder physiologisch verträglichen Salze zur Behandlung und/oder Prävention von Bluthochdruck, Herzhypertrophie, Herzinsuffizienz, koronaren Herzerkrankungen, einer endothelialen Dysfunktion oder endothelialer Schädigungen oder von Diabetis mellitus.

14. Verbindungen der Formel (I) gemäß einem oder mehrere des Ansprüche 1-6 und/oder ihre Solvate oder physiologish verträglichen Salze zur Behandlung und/oder Prävention von koronaren Herzerkrankungen, **dadurch gekennzeichnet, daß** es sich bei der koronaren Herzerkrankung um Angina Pectoris handelt.

## Claims

1. A compound of the formula (I) 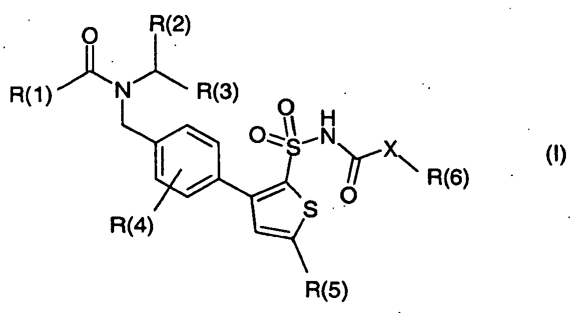
in which the indicated radicals have the following meaning:
R(1) is
1. (C₁-C₅)-alkyl, unsubstituted or substituted by a radical from the series NH₂, halogen, O-(C₁-C₃)-alkyl, CO-O-(C₁-C₂)-alkyl and CO₂H;
2. (C₃-C₈)-cycloalkyl;
3. (C₁-C₃)-alkyl-(C₃-C₈)-cycloalkyl;
4. (C₆-C₁₀)-aryl, unsubstituted or substituted by a radical from the series halogen and O-(C₁-C₃)-alkyl;
5. (C₁-C₃)-alkyl-(C₆-C₁₀)-aryl, where the aryl radical is unsubstituted or substituted by a radical from the series halogen and O-(C₁-C₃)-alkyl;
6. (C₁-C₅)-heteroaryl; and
7. (C₁-C₃)-alkyl-(C₁-C₅)-heteroaryl;
R(2) is
1. hydrogen;
2. (C₁-C₆)-alkyl, unsubstituted or substituted by a radical from the series halogen and O-(C₁-C₃)-alkyl;
3. (C₃-C₈)-cycloalkyl;
4. (C₁-C₃)-alkyl-(C₃-C₈)-cycloalkyl;
5. (C₆-C₁₀)-aryl, unsubstituted or substituted by a radical from the series halogen, O-(C₁-C₃)-alkyl and CO-O-(C₁-C₃)-alkyl; and
6. (C₁-C₃)-alkyl-(C₆-C₁₀)-aryl, unsubstituted or substituted by a radical from the series halogen and O-(C₁-C₃)-alkyl;
R(3) is
1. hydrogen;
2. COOH; and
3. COO-(C₁-C₄)-alkyl;
R(4) is
1. hydrogen;
2. halogen; and
3. (C₁-C₄)-alkyl;
R(5) is
1. hydrogen, and
2. (C₁-C₆)-alkyl;
R(6) is
1. hydrogen;
2. (C₁-C₆)-alkyl;
3. (C₁-C₃)-alkyl-(C₃-C₈)-cycloalkyl; and
4. (C₂-C₆)-alkenyl;
X is
1. oxygen, and
2. NH;
in all the stereoisomeric forms thereof, and mixtures thereof in all ratios, the solvates thereof and the physiologically tolerated salts thereof.

2. A compound of the formula (I) as claimed in claim 1 in which:
R(1) is
1. (C₁-C₅)-alkyl, unsubstituted or substituted by a radical from the series NH₂, halogen, O-(C₁-C₃)-alkyl, CO-O-(C₁-C₃)-alkyl and CO₂H;
2. (C₃-C₆)-cycloalkyl;
3. (C₁-C₃)-alkyl-(C₃-C₆)-cycloalkyl;
4. (C₆-C₁₀)-aryl; unsubstituted or substituted by a radical from the series halogen and O-(C₁-C₃)-alkyl;
5. (C₁-C₃)-alkyl-(C₆-C₁₀)-aryl, where the aryl radical is unsubstituted or substituted by a radical from the series halogen and O-(C₁-C₃)-alkyl;
6. (C₃-C₅)-heteroaryl; and
7. (C₁-C₃)-alkyl-(C₃-C₅)-heteroaryl;
R(2) is
1. hydrogen;
2. (C₁-C₆)-alkyl, unsubstituted or substituted by a radical from the series halogen and O-(C₁-C₃)-alkyl;
3. (C₃-C₆)-cycloalkyl;
4. (C₁-C₃)-alkyl-(C₃-C₆)-cycloalkyl;
5. (C₆-C₁₀)-aryl, unsubstituted or substituted by a radical from the series halogen, O-(C₁-C₃)-alkyl and CO-O-(C₁-C₃)-alkyl; and
6. (C₁-C₃)-alkyl-(C₆-C₁₀)-aryl, unsubstituted or substituted by a radical from the series halogen and O-(C₁-C₃)-alkyl;
R(3) is
1. hydrogen;
2. COOH; and
3. COO-(C₁-C₄)-alkyl;
R(4) is
1. hydrogen;
2. halogen; and
3. (C₁-C₄)-alkyl;
R(5) is
1. hydrogen, and
2. (C₁-C₄)-alkyl;
R(6) is
1. hydrogen;
2. (C₁-C₄)-alkyl;
3. (C₁-C₃)-alkyl-(C₃-C₆)-cycloalkyl; and
4. (C₃-C₅)-alkenyl;
X is
1. oxygen, and
2. NH;
in all the stereoisomeric forms thereof, and mixtures thereof in all ratios, the solvates thereof and the physiologically tolerated salts thereof.

3. A compound of the formula (I) as claimed in claim 1 or 2, in which:
R(1) is
1. (C₁-C₃)-alkyl, unsubstituted or substituted by a radical from the series fluorine, methoxy, ethoxy, CO-O-(C₁-C₃)-alkyl and CO₂H;
2. (C₁-C₃)-alkyl-cyclohexyl;
3. phenyl, substituted or unsubstituted by a radical from the series fluorine and methoxy;
4. (C₁-C₃)-alkyl-phenyl, where the phenyl radical is unsubstituted or substituted by a radical from the series fluorine and methoxy;
5. furanyl, thienyl or pyridyl;
R(2) is
1. hydrogen;
2. (C₁-C₆)-alkyl, unsubstituted or substituted by a radical from the series fluorine, methoxy and ethoxy;
3. phenyl, unsubstituted or substituted by a radical from the series fluorine and methoxy;
4. (C₁-C₆)-cycloalkyl;
R(4) is
1. hydrogen;
2. methyl; and
3. chlorine;
R(5) is (C₁-C₄)-alkyl;
R(6) is (C₁-C₄)-alkyl;
and the radicals R(3) and X are as defined above, in all the stereoisomeric forms thereof, and mixtures thereof, the solvates thereof and the physiologically tolerated salts thereof.

4. A compound of the formula (I) as claimed in one or more of claims 1 to 3, in which:
R(1) is
1. (C₁-C₃)-alkyl, unsubstituted or substituted by a radical from the series fluorine, methoxy, ethoxy, CO-O-(C₁-C₃)-alkyl and CO₂H;
2. (C₁-C₃)-alkyl-cyclohexyl;
3. phenyl, substituted or unsubstituted by a radical from the series fluorine and methoxy;
4. (C₁-C₃)-alkyl-phenyl, where the phenyl radical is unsubstituted or substituted by a radical from the series fluorine and methoxy;
5. furanyl, thienyl or pyridyl;
R(2) is
1. hydrogen;
2. (C₁-C₆)-alkyl, unsubstituted or substituted by a radical from the series fluorine, methoxy and ethoxy;
3. phenyl, unsubstituted or substituted by a radical from the series fluorine and methoxy;
4. cyclopropyl or cyclohexyl;
R(4) is
1. hydrogen;
2. methyl; and
3. chlorine;
R(5) is propyl and butyl, in particular n-propyl, isopropyl and 2-isobutyl;
R(6) is methyl, ethyl, n-propyl, isopropyl, n-butyl and isobutyl;
and the radicals R(3) and X are as defined above, in all the stereoisomeric forms thereof, and mixtures thereof, the solvates thereof and the physiologically tolerated salts thereof.

5. A compound of the formula (I) as claimed in one or more of claims 1 to 4, which is a compound of the formula (II) 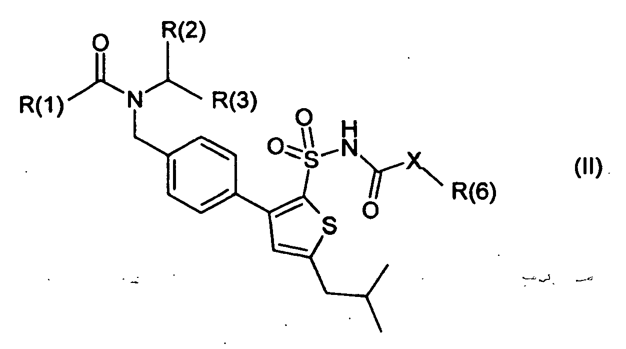
in which the radicals R(1), R(2), R(3), R(6) and X have the meaning given in one or more of claims 1 to 4, in all the stereoisomeric forms thereof, and mixtures thereof, the solvates thereof and the physiologically tolerated salts thereof.

6. A compound of the formula (I) as claimed in one or more of claims 1 to 5, which is one of the following
methyl 2-*N*-benzoyl-2-*N*-[4-[2-(*n*-*b*utyloxycarbonylsulfonamido)-5-isobutyl-3-thienyl]benzyl]amino-3-methyl-L-butyrate;
ethyl 2-*N*-benzoyl-2-*N*-[4-[2-(*n*-butyloxycarbonylsulfonamido)-5-isobutyl-3-thienyl]benzyl]amino-3-methyl-L-butyrate;
2-*N*-benzoyl-2-*N*-[4-[2-(*n*-butyloxycarbonylsulfonamido)-5-isobutyl-3-thienyl]benzyl]amino-3-methyl-L-butyric acid;
methyl 2-*N*-benzoyl-2-*N*-[4-[2-(ethylaminocarbonylsulfonamido)-5-isobutyl-3-thienyl]benzyl]amino-3-methyl-L-butyrate;
ethyl 2-*N*-benzoyl-2-*N*-[4-[2-(ethylaminocarbonylsulfonamido)-5-isobutyl-3-thienyl]benzyl]amino-3-methyl-L-butyrate;
2-*N*-benzoyl-2-*N*-[4-[2-(ethylaminocarbonylsulfonamido)-5-isobutyl-3-thienyl]benzyl]amino-3-methyl-L-butyric acid;
methyl 2-*N*-acetyl-2-*N*-[4-[2-(*n*-butyloxycarbonylsulfonamido)-5-isobutyl-3-thienyl]benzyl]amino-3-methyl-L-butyrate;
2-*N*-acetyl-2-*N*-[4-[2-(*n*-butyloxycarbonylsulfonamido)-5-isobutyl-3-thienyl]benzyl]amino-3-methyl-L-butyric acid;
methyl 2-*N*-acetyl-2-*N*-[4-[2-(ethylaminocarbonylsulfonamido)-5-isobutyl-3-thienyl]benzyl]amino-3-methyl-L-butyrate;
ethyl 2-*N*-acetyl-2-*N*-[4-[2-(ethylaminocarbonylsulfonamido)-5-isobutyl-3-thienyl]benzy]amino-3-methyl-L-butyrate;
2-*N*-acetyl-2-*N*-[4-[2-(ethylaminocarbonylsulfonamido)-5-isobutyl-3-thienyl]benzyl]amino-3-methyl-L-butyric acid;
methyl 2-*N*-(2-furanoyl)-2-*N*-[4-[2-(*n*-butyloxycarbonylsulfonamido)-5-isobutyl-3-thienyl]benzyl]amino-3-methyl-L-butyrate;
2-*N*-(2-furanoyl)-2-*N*-[4-[2-(*n*-butyloxycarbonylsulfonamido)-5-isobutyl-3-thienyl]benzyl]amino-3-methyl-L-butyric acid;
methyl 2-*N*-(2-furanoyl)-2-*N*-[4-[2-(methylaminocarbonylsulfonamido)-5-isobutyl-3-thienyl]benzyl]amino-3-methyl-L-butyrate;
2-*N*-(2-furanoyl)-2-*N*-[4-[2-(methylaminocarbonylsulfonamido)-5-isobutyl-3-thienyl]benzyl]amino-3-methyl-L-butyric acid;
methyl 2-*N*-(3-methoxycarbonylpropionyl)-2-*N*-[4-[2-(*n-*butyloxycarbonylsulfonamido)-5-isobutyl-3-thienyl]benzyl]amino-3-methyl-L-butyrate;
2-*N*-(3-carboxypropionyl)-2-*N*-[4-[2-(*n*-butyloxycarbonylsulfonamido)-5-isobutyl-3-thienyl]benzyl]amino-3-methyl-L-butyric acid;
methyl 2-*N*-(3-methoxycarbonylpropionyl)-2-*N*-[4-[2-(ethylaminocarbonylsulfonamido)-5-isobutyl-3-thienyl]benzyl]amino-3-methyl-L-butyrate;
2-*N*-(3-carboxypropionyl)-2-*N*-[4-[2-(ethylaminocarbonylsulfonamido)-5-isobutyl-3-thienyl]benzyl]amino-3-methyl-L-butyric acid;
methyl 2-*N*-benzoyl-2-*N*-[4-[2-(ethylaminocarbonylsulfonamido)-5-isobutyl-3-thienyl]benzyl]amino-2S-phenylacetate;
methyl 2-*N*-acetyl-2-*N*-[4-[2-(ethylaminocarbonylsulfonamido)-5-isobutyl-3-thienyl]benzyl]amino-2S-phenylacetate;
methyl 2-*N*-benzoyl-2-*N*-[4-[2-(ethylaminocarbonylsulfonamido)-5-isobutyl-3-thienyl]benzyl]amino-2S-cyclohexylacetate;
methyl 2-*N*-acetyl-2-*N*-[4-[2-(ethylaminocarbonylsulfonamido)-5-isobutyl-3-thienyl]benzyl]amino-2S-cyclohexylacetate;
2-cyclohexyl-*N*-(2-methoxyethyl)-*N*-[4-[2-(*n*-butyloxycarbonylsulfonamido)-5-isobutyl-3-thienyl]benzyl]acetamide;
2-cyclohexyl-*N*-(2-methoxyethyl)-*N*-[4-[2-(ethylaminocarbonylsulfonamido)-5-isobutyl-3-thienyl]benzyl]acetamide;
2-cyclohexyl-*N*-cyclopropyl-*N*-[4-[2-(*n*-butyloxycarbonylsulfonamido)-5-isobutyl-3-thienyl]benzyl]acetamide; or
2-cyclohexyl-N-cyclopropyl-N-[4-[2-(ethylaminocarbonylsulfonamido)-5-isobutyl-3-thienyl]benzyl]acetamide;
the solvates thereof and the physiologically tolerated salts thereof.

7. A process for preparing a compound of the formula (I) as claimed in one or more of claims 1 to 6, which comprises
a) reacting thiophene-3-boronic acids of the formula (III) 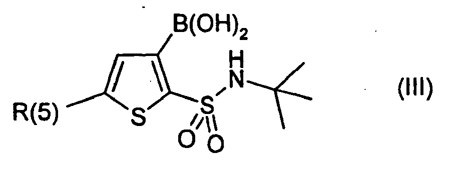
with p-bromobenzaldehydes of the formula (IV) 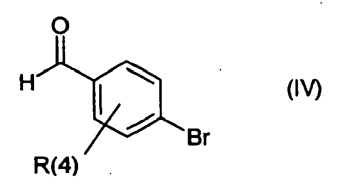
to give compounds of the formula (V) 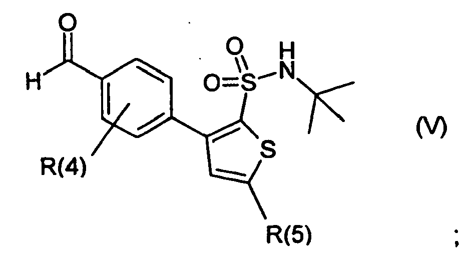
b) reacting the compounds of the formula (V) with primary amino compounds of the formula (VI) 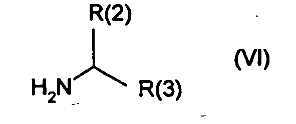
to give compounds of the formula (VII) 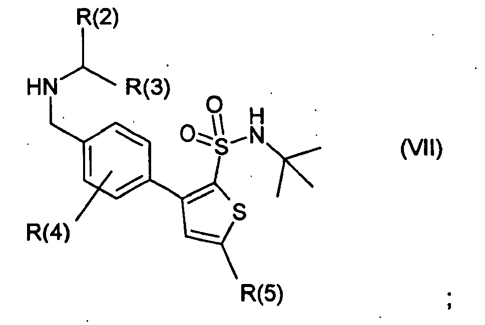
c) reacting the compounds of the formula (VII) with acyl chlorides of the type R(1)-COCl to give amides of the formula (VIII) 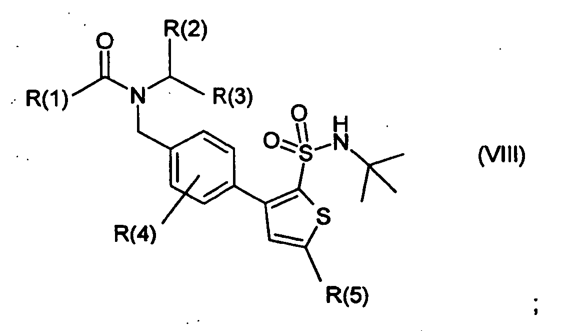
d) converting the compounds of the formula (VIII), by eliminating the *tert-*butyl protecting group, into sulfonamides of the formula (IX), and 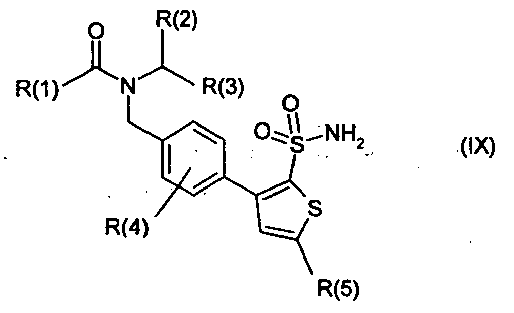
e) converting the compounds of the formula (IX), by reaction with R(6)-substituted chloroformic esters, into the corresponding sulfonylurethanes of the formula (I) (X is O), or, by reaction with R(6)-substituted isocyanates, into the corresponding sulfonylureas of the formula (I) (X is NH), with R(1), R(2), R(3), R(4), R(5) and R(6) being defined as in claims 1 to 6.

8. A compound of the formula (I) as claimed in one or more of claims 1 to 7, and/or the solvates thereof or the physiologically tolerated salts thereof, for use as a pharmaceutical.

9. A pharmaceutical preparation, which has an effective content of a compound of the formula (I) as claimed in one or more of claims 1 to 6 and/or a solvate or a physiologically tolerated salt thereof.

10. A pharmaceutical preparation as claimed in claim 9, which has an effective content of an additional active compound.

11. A compound of the formula (I) as claimed in one or more of claims 1 to 6 and/or a solvate or a physiologically tolerated salt thereof for stimulating angiotensin-(1-7) receptors.

12. A compound of the formula (I) as claimed in one or more of claims 1 to 6 and/or a solvate or a physiologically tolerated salt thereof, for treating and/or preventing diseases the primary or secondary cause of which, or at least a primary or secondary component cause of which, is a reduced production and/or release of the vasorelaxing, antithrombotic and cardioprotective messengers cyclic 3',5'-guanosine monophoshate (cGMP) and nitrogen monoxide (NO).

13. A compound of the formula (I) as claimed in one or more of claims 1 to 6 and/or a solvate or a physiologically tolerated salt thereof, for treating and/or preventing hypertension, cardiac hypertrophy, cardiac insufficiency, coronary heart diseases, endothelial dysfunction or endothelial damage or Diabetes mellitus.

14. A compound of the formula (I) as claimed in one or more of claims 1 to 6 and/or a solvate or physiologically tolerated salt thereof for treating and/or preventing coronary heart diseases, wherein the coronary heart disease is Angina pectoris.

## Revendications

1. Composés de formule (I) 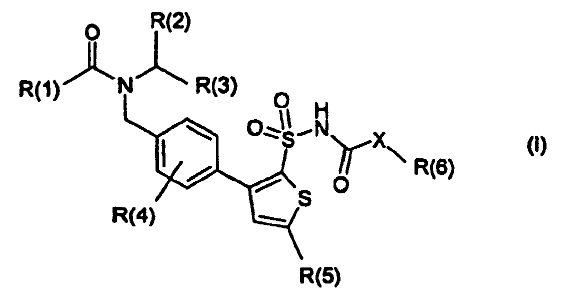
dans laquelle les radicaux indiqués ont la signification suivante :
R(1)
1. alkyle en C₁ à C₅, non substitué ou substitué par un radical de la série NH₂, halogène, O-alkyle en C₁ à C₃, CO-O-alkyle en C₁ à C₃ et CO₂H ;
2. cycloalkyle en C₃ à C₈ ;
3. (alkyle en C₁ à C₃) (cycloalkyle en C₃ à C₈) ;
4. aryle en C₆ à C₁₀, non substitué ou substitué par un radical de la série halogène et O-alkyle en C₁ à C₃ ;
5. (alkyle en C₁ à C₃) (aryle en C₆ à C₁₀), le radical aryle étant non substitué ou substitué par un radical de la série halogène et O-alkyle en C₁ à C₃ ;
6. hétéroaryle en C₁ à C₅ ; et
7. (alkyle en C₁ à C₃) (hétéroaryle en C₁ à C₅) ;
R(2)
1. hydrogène ;
2. alkyle en C₁ à C₆, non substitué ou substitué par un radical de la série halogène et O-alkyle en C₁ à C₃ ;
3. cycloalkyle en C₃ à C₈ ;
4. (alkyle en C₁ à C₃) (cycloalkyle en C₃ à C₈) ;
5. aryle en C₆ à C₁₀, non substitué ou substitué par un radical de la série halogène, O-alkyle en C₁ à C₃ et CO-O-alkyle en C₁ à C₃ ; et
6. (alkyle en C₁ à C₃) (aryle en C₆ à C₁₀), non substitué ou substitué par un radical de la série halogène et O-alkyle en C₁ à C₃ ;
R(3)
1. hydrogène ;
2. COOH ; et
3. COO-alkyle en C₁ à C₄ ;
R(4)
1. hydrogène ;
2. halogène ; et
3. alkyle en C₁ à C₄ ;
R(5)
1. hydrogène et
2. alkyle en C₁ à C₆ ;
R(6)
1. hydrogène ;
2. alkyle en C₁ à C₆ ;
3. (alkyle en C₁ à C₃) (cycloalkyle en C₃ à C₈) ; et
4. alcényle en C₂ à C₆ ;
X
1. oxygène et
2. NH ;
dans toutes leurs formes stéréo-isomères et tous les mélanges de ceux-ci, dans tous les rapports, leurs solvates et leurs sels physiologiquement acceptables.

2. Composés de formule (I) selon la revendication 1, présentant les significations :
R(1)
1. alkyle en C₁ à C₅, non substitué ou substitué par un radical de la série NH₂, halogène, O-alkyle en C₁ à C₃, CO-O-alkyle en C₁ à C₃ et CO₂H ;
2. cycloalkyle en C₃ à C₆ ;
3. (alkyle en C₁ à C₃) (cycloalkyle en C₃ à C₆) ;
4. aryle en C₆ à C₁₀, non substitué ou substitué par un radical de la série halogène et O-alkyle en C₁ à C₃ ;
5. (alkyle en C₁ à C₃) (aryle en C₆ à C₁₀), le radical aryle étant non substitué ou substitué par un radical de la série halogène et O-alkyle en C₁ à C₃ ;
6. hétéroaryle en C₃ à C₅ ; et
7. (alkyle en C₁ à C₃) (hétéroaryle en C₃ à C₅) ;
R(2)
1. hydrogène ;
2. alkyle en C₁ à C₆, non substitué ou substitué par un radical de la série halogène et O-alkyle en C₁ à C₃ ;
3. cycloalkyle en C₃ à C₆ ;
4. (alkyle en C₁ à C₃) (cycloalkyle C₃ à C₆) ;
5. aryle en C₆ à C₁₀, non substitué ou substitué par un radical de la série halogène, O-alkyle en C₁ à C₃ et CO-O-alkyle en C₁ à C₃ ; et
6. (alkyle en C₁ à C₃) (aryle en C₆ à C₁₀), non substitué ou substitué par un radical de la série halogène et O-alkyle en C₁ à C₃ ;
R(3)
1. hydrogène ;
2. COOH ; et
3. COO-alkyle en C₁ à C₄ ;
R(4)
1. hydrogène ;
2. halogène ; et
3. alkyle en C₁ à C₄ ;
R(5)
1. hydrogène et
2. alkyle en C₁ à C₄ ;
R(6)
1. hydrogène ;
2. alkyle en C₁ à C₄ ;
3. (alkyle en C₁ à C₃) (cycloalkyle en C₃ à C₆) ; et
4. alcényle en C₃ à C₅ ;
X
1. oxygène et
2. NH ;
dans toutes leurs formes stéréo-isomères et tous les mélanges de ceux-ci, dans tous les rapports, leurs solvates et leurs sels physiologiquement acceptables.

3. Composés de formule (I) selon la revendication 1 ou 2, présentant les significations:
R(1)
1. alkyle en C₁ à C₃, non substitué ou substitué par un radical de la série fluor, méthoxy, éthoxy, CO-O-alkyle en C₁ à C₃ et CO₂H ;
2. (alkyle en C₁ à C₃)cyclohexyle ;
3. phényle, substitué ou non substitué par un radical de la série fluor et méthoxy ;
4. (alkyle en C₁ à C₃)phényle, le radical phényle étant non substitué ou substitué par un radical de la série fluor et méthoxy ;
5. furannyle, thiényle ou pyridyle ;
R(2)
1. hydrogène ;
2. alkyle en C₁ à C₆, non substitué ou substitué par un radical de la série fluor, méthoxy et éthoxy ;
3. phényle, non substitué ou substitué par un radical de la série fluor et méthoxy ;
4. cycloalkyle en C₁ à C₆ ;
R(4)
1. hydrogène ;
2. méthyle ; et
3. chlore ;
R(5) alkyle en C₁ à C₄ ;
R (6) alkyle en C₁ à C₄ ;
et les radicaux R(3) et X étant définis comme dans les revendications précédentes dans toutes leurs formes stéréo-isomères et tous les mélanges de ceux-ci, leurs solvates et leurs sels physiologiquement acceptables.

4. Composés de formule (I) selon l'une ou plusieurs des revendications 1 à 3, présentant les significations :
R(1)
1. alkyle en C₁ à C₃, non substitué ou substitué par un radical de la série fluor, méthoxy, éthoxy, CO-O-alkyle en C₁ à C₃ et CO₂H ;
2. (alkyle en C₁ à C₃) cyclohexyle ;
3. phényle, substitué ou non substitué par un radical de la série fluor et méthoxy ;
4. (alkyle en C₁ à C₃) phényle, le radical phényle étant non substitué ou substitué par un radical de la série fluor et méthoxy ;
5. furannyle, thiényle ou pyridyle ;
R(2)
1. hydrogène ;
2. alkyle en C₁ à C₆, non substitué ou substitué par un radical de la série fluor, méthoxy et éthoxy ;
3. phényle, non substitué ou substitué par un radical de la série fluor et méthoxy ;
4. cyclopropyle ou cyclohexyle ;
R(4)
1. hydrogène ;
2. méthyle ; et
3. chlore ;
R(5) propyle et butyle, en particulier n-propyle, iso-propyle et 2-iso-butyle ;
R(6) méthyle, éthyle, n-propyle, iso-propyle, n-butyle et iso-butyle ;
et les radicaux R(3) et X étant définis comme dans les revendications précédentes dans toutes leurs formes stéréo-isomères et tous les mélanges de ceux-ci, leurs solvates et leurs sels physiologiquement acceptables.

5. Composés de formule (I) selon l'une ou plusieurs des revendications 1 à 4, ces composés présentant la formule (II), 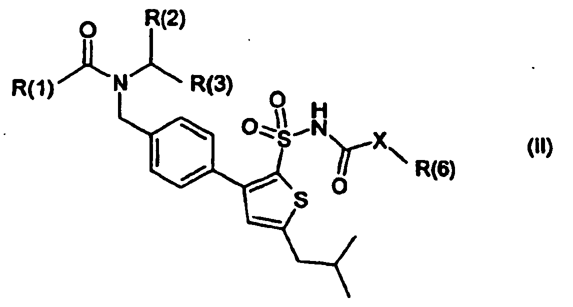
dans laquelle les radicaux R(1), R(2), R(3), R(6) et X ont la signification indiquée dans l'une ou plusieurs des revendications 1 à 4, dans toutes leurs formes stéréo-isomères et tous les mélanges de ceux-ci, leurs solvates et leurs sels physiologiquement acceptables.

6. Composés de formule (I) selon l'une ou plusieurs des revendications 1 à 5, **caractérisés en ce qu'**il s'agit des composés
ester méthylique de l'acide 2-N-benzoyl-2-N-[4-[2-(n-butyloxycarbonylsulfonamido)-5-isobutyl-3-thiényl]benzyl]amino-3-méthyl-L-butyrique;
ester éthylique de l'acide 2-N-benzoyl-2-N-[4-[2-(n-butyloxycarbonylsulfonamido)-5-isobutyl-3-thiényl]benzyl]amino-3-méthyl-L-butyrique ;
acide 2-N-benzoyl-2-N-[4-[2-(n-butyloxycarbonylsulfonamido)-5-isobutyl-3-thiényl]benzyl]amino-3-méthyl-L-butyrique ;
ester méthylique de l'acide 2-N-benzoyl-2-N-[4-[2-(éthylaminocarbonylsulfonamido)-5-isobutyl-3-thiényl]-benzyl]amino-3-méthyl-L-butyrique ;
ester éthylique de l'acide 2-N-benzoyl-2-N-[4-[2-(éthylaminocarbonylsulfonamido)-5-isobutyl-3-thiényl]benzyl]amino-3-méthyl-L-butyrique ;
acide 2-N-benzoyl-2-N-[4-[2-(éthylaminocarbonylsulfonamido)-5-isobutyl-3-thiényl]benzyl]amino-3-méthyl-L-butyrique ;
ester méthylique de l'acide 2-N-acétyl-2-N-[4-[2-(n-butyloxycarbonylsulfonamido)-5-isobutyl-3-thiényl]benzyl]amino-3-méthyl-L-butyrique ;
acide 2-N-acétyl-2-N-[4-[2-(n-butyloxycarbonylsulfonamido)-5-isobutyl-3-thiényl]benzyl]amino-3-méthyl-L-butyrique ;
ester méthylique de l'acide 2-N-acétyl-2-N-[4-[2-(éthylaminocarbonylsulfonamido)-5-isobutyl-3-thiényl]benzyl]amino-3-méthyl-L-butyrique;
ester éthylique de l'acide 2-N-acétyl-2-N-[4-[2-(éthylaminocarbonylsulfonamido)-5-isobutyl-3-thiényl]benzyl]amino-3-méthyl-L-butyrique ;
acide 2-N-acétyl-2-N-[4-[2-(éthylaminocarbonylsulfonamido)-5-isobutyl-3-thiényl]benzyl]amino-3-méthyl-L-butyrique ;
ester méthylique de l'acide 2-N-(2-furannoyl)-2-N-[4-[2-(n-butyloxycarbonylsulfonamido)-5-isobutyl-3-thiényl]benzyl]amino-3-méthyl-L-butyrique ;
acide 2-N-(2-furannoyl)-2-N-[4-[2-(n-butyloxycarbonylsulfonamido)-5-isobutyl-3-thiényl]benzyl]amino-3-méthyl-L-butyrique ;
ester méthylique de l'acide 2-N-(2-furannoyl)-2-N-[4-[2-(méthylaminocarbonylsulfonamido)-5-isobutyl-3-thiényl]benzyl]amino-3-méthyl-L-butyrique ;
acide 2-N-(2-furannoyl)-2-N-[4-[2-(méthylaminocarbonylsulfonamido)-5-isobutyl-3-thiényl]benzyl]amino-3-méthyl-L-butyrique ;
ester méthylique de l'acide 2-N-(3-méthoxycarbonylpropionyl)-2-N-[4-[2-(n-butyloxycarbonylsulfonamido)-5-isobutyl-3-thiényl]benzyl]amino-3-méthyl-L-butyrique ;
acide 2-N-(3-carboxypropionyl)-2-N-[4-[2-(n-butyloxycarbonylsulfonamido)-5-isobutyl-3-thiényl]benzyl]amino-3-méthyl-L-butyrique ;
ester méthylique de l'acide 2-N-(3-méthoxycarbonylpropionyl)-2-N-[4-[2-(éthylaminocarbonylsulfonamido)-5-isobutyl-3-thiényl]benzyl]amino-3-méthyl-L-butyrique ;
acide 2-N-(3-carboxypropionyl)-2-N-[4-[2-(éthylaminocarbonylsulfonamido)-5-isobutyl-3-thiényl]benzyl]amino-3-méthyl-L-butyrique ;
ester méthylique de l'acide 2-N-benzoyl-2-N-[4-[2-(éthylaminocarbonylsulfonamido)-5-isobutyl-3-thiényl]benzyl]amino-2S-phénylacétique ;
ester méthylique de l'acide 2-N-acétyl-2-N-[4-[2-(éthylaminocarbonylsulfonamido)-5-isobutyl-3-thiényl]benzyl]amino-2S-phénylacétique ;
ester méthylique de l'acide 2-N-benzoyl-2-N-[4-[2-(éthylaminocarbonylsulfonamido)-5-isobutyl-3-thiényl]benzyl]amino-2S-cyclohexylacétique ;
ester méthylique de l'acide 2-N-acétyl-2-N-[4-[2-(éthylaminocarbonylsulfonamido)-5-isobutyl-3-thiényl]benzyl]amino-2S-cyclohexylacétique ;
2-cyclohexyl-N-(2-méthoxyéthyl)-N-[4-[2-(n-butyloxycarbonylsulfonamido)-5-isobutyl-3-thiényl]benzyl]acétamide ;
2-cyclohexyl-N-(2-méthoxyéthyl)-N-[4-[2-(éthylaminocarbonylsulfonamido)-5-isobutyl-3-thiényl]benzyl]acétamide ;
2-cyclohexyl-N-cyclopropyl-N-[4-[2-(n-butyloxycarbonylsulfonamido)-5-isobutyl-3-thiényl]benzyl]acétamide ; ou
2-cyclohexyl-N-cyclopropyl-N-[4-[2-(éthylaminocarbonylsulfonamido)-5-isobutyl-3-thiényl]benzyl]acétamide ;
ainsi que leurs solvates et leurs sels physiologiquement acceptables.

7. Procédé pour la préparation de composés de formule (I) selon l'une ou plusieurs des revendications 1 à 6, **caractérisé en ce qu'**on transforme
a) des acides thiophène-3-boroniques de formule (III), 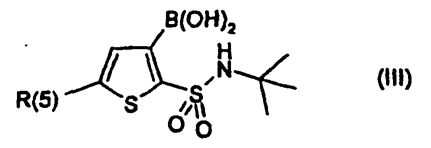
avec des p-bromobenzaldéhydes de formule (IV), 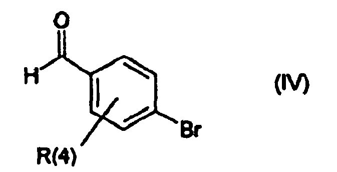
en composés de formule (V), 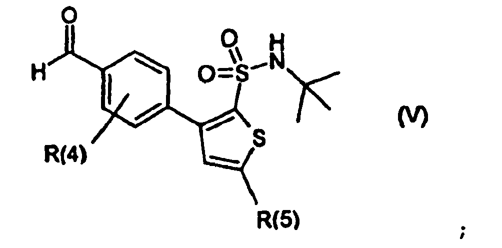
b) on transforme les composés de formule (V) avec des composés amino primaires de formule (VI) 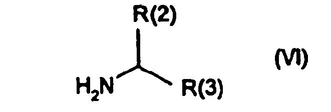
en composés de formule (VII), 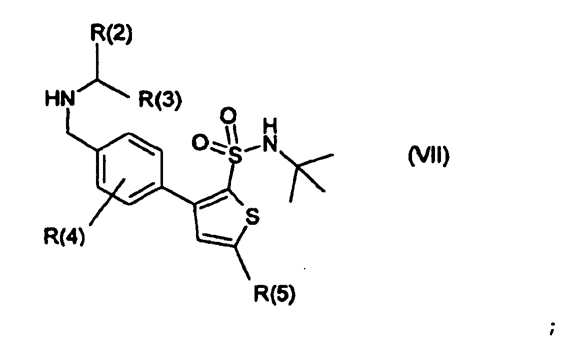
c) on transforme les composés de formule (VII) avec des chlorures d'acyle du type R(1)-COCl en amides de formule (VIII) 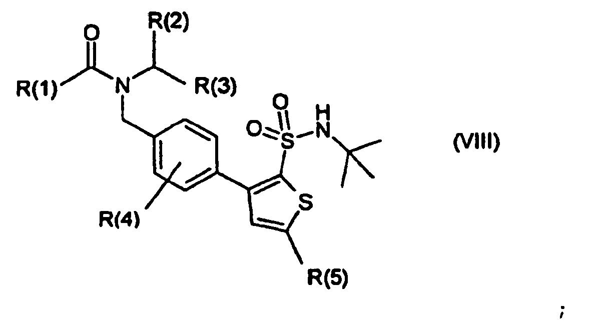
d) on transforme les composés de formule (VIII) par dissociation du groupe de protection tert-butyle en sulfonamides de formule (IX) 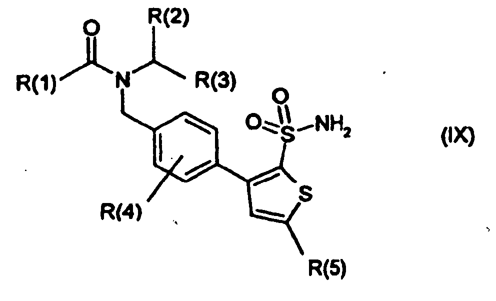 et
e) on transforme les composés de (IX) par transformation avec des esters de l'acide chloroformique substitués par R(6) en sulfonyluréthanes correspondants de formule (I) (X représentant O), ou par transformation avec des isocyanates substitués par R(6) en sulfonylurées correspondantes de formule (I) (X représentant NH), où R(1), R(2), R(3), R(4), R(5) et R(6) sont définis comme dans les revendications 1 à 6.

8. Composés de formule (I) selon l'une ou plusieurs des revendications 1 à 7 et/ou leurs solvates ou sels physiologiquement acceptables destinés à une utilisation comme médicament.

9. Préparation pharmaceutique, **caractérisée par** une teneur active d'un composé de formule (I) selon l'une ou plusieurs des revendications 1 à 6 ou un solvate ou un sel physiologiquement acceptable de celui-ci.

10. Préparation pharmaceutique selon la revendication 9, **caractérisée par** une teneur active en une autre substance active.

11. Composés de formule (I) selon l'une ou plusieurs des revendications 1 à 6 et/ou leurs solvates ou sels physiologiquement acceptables pour la stimulation des récepteurs de l'angiotensine (1-7).

12. Composés de formule (I) selon l'une ou plusieurs des revendications 1 à 6 et/ou leurs solvates ou sels physiologiquement acceptables pour la thérapie et/ou la prévention de maladies, qui sont provoquées ou du moins co-provoquées de manière primaire ou secondaire par une production réduite et/ou une libération réduite des substances messagères vasorelaxantes, antithrombotiques et cardioprotectives 3'5'-guanosine monophosphate cyclique (cGMP) et monoxyde d'azote (NO).

13. Composés de formule (I) selon l'une ou plusieurs des revendications 1 à 6 et/ou leurs solvates ou sels physiologiquement acceptables pour le traitement et/ou la prévention de l'hypertension sanguine, de l'hypertrophie cardiaque, de l'insuffisance cardiaque, des maladies coronaro-cardiaques, d'un dysfonctionnement endothélial ou de dommages endothéliaux ou du diabète mellitus.

14. Composés de formule (I) selon l'une ou plusieurs des revendications 1 à 6 et/ou leurs solvates ou sels physiologiquement acceptables pour le traitement et/ou la prévention de maladies coronaro-cardiaques, **caractérisés en ce qu'**il s'agit, pour les maladies coronaro-cardiaques de l'angine de poitrine.
